# EUROPÄISCHE PATENTSCHRIFT

(11) **EP 2 052 055 B1**
(45) Veröffentlichungstag und Bekanntmachung des Hinweises auf die Patenterteilung: **06.07.2011**
(21) Anmeldenummer: 07786156.5
(22) Anmeldetag: 18.07.2007
(51) Int. Cl.: C09K 19/30, C09K 19/32, C09K 19/34, C09K 19/42

(54) **CYCLOHEXEN-VERBINDUNGEN FÜR FLÜSSIGKRISTALLINE MISCHUNGEN**
CYCLOHEXENE COMPOUNDS FOR LIQUID-CRYSTALLINE MIXTURES
COMPOSÉS DE CYCLOHEXÈNE POUR MÉLANGES LIQUIDES CRISTALLINS

(30) Priorität: 16.08.2006 DE 102006038150
(43) Veröffentlichungstag der Anmeldung: 29.04.2009
(73) Patentinhaber: Merck Patent GmbH, 64293 Darmstadt (DE)
(72) Erfinder: LIETZAU, Lars, 64295 Darmstadt (DE); CZANTA, Markus, 64289 Darmstadt (DE); MANABE, Atsutaka, 64625 Bensheim (DE); JAEHRLING, Kai, 64686 Lautertal (DE)
(86) Internationale Anmeldenummer: PCT/EP2007/006382
(87) Internationale Veröffentlichungsnummer: WO 2008/019743

(56) Entgegenhaltungen:
- EP-A- 1 029 842
- EP-A- 1 482 018
- EP-A1- 0 786 445
- EP-A1- 0 844 229
- EP-A1- 1 053 996
- EP-A2- 1 081 123
- DE-A1- 10 013 681

## Beschreibung

Die Erfindung betrifft 1,4-substituierte Cyclohexen-Derivate und 2-Fluorocyclohexen-Derivate sowie ihre Verwendung als Komponente(n) in flüssigkristallinen Medien. Darüber hinaus betrifft die vorliegende Erfindung Flüssigkristall- und elektrooptische Anzeigeelemente, welche die erfindungsgemäßen, flüssigkristallinen Medien enthalten. Die erfindungsgemäßen Verbindungen weisen eine Difluormethylenoxy-Gruppe in einer bestimmten Anordnung auf.

In den vergangenen Jahren wurden die Anwendungsgebiete für flüssigkristalline Verbindungen auf verschiedene Arten von Anzeigevorrichtungen, elektrooptische Geräte, elektronische Komponenten, Sensoren, etc. erheblich ausgeweitet. Aus diesem Grund wurden eine Reihe verschiedener Strukturen vorgeschlagen, insbesondere auf dem Gebiet der nematischen Flüssigkristalle. Die nematischen Flüssigkristallmischungen haben bisher die breiteste Anwendung in flachen Anzeigevorrichtungen gefunden. Sie wurden besonders in passiven TN- oder STN-Matrixanzeigen oder Systemen mit einer TFT-Aktivmatrix eingesetzt.

Die erfindungsgemäßen, flüssigkristallinen Verbindungen können als Komponente(n) flüssigkristalliner Medien verwendet werden, insbesondere für Displays, die auf dem Prinzip der verdrillten Zelle, dem Guest-Host-Effekt, dem Effekt der Deformation aufgerichteter Phasen DAP oder ECB (electrically controlled birefringence), dem IPS-Effekt (in-plane switching) oder dem Effekt der dynamischen Streuung beruhen.

Die Verwendung bestimmter Derivate mit einer Difluormethylenoxy-Brücke (-CF₂O-) als flüssigkristalline Substanzen ist dem Fachmann bekannt. In den Druckschriften EP 0844229 A1 und EP 1029842 A2 werden Substanzen mit einem Cyclohexenring und einer CF₂O-Gruppe offenbart. Allerdings ist in den Verbindungen die Doppelbindung des Cyclohexenrings direkt mit der Difluormethyleneinheit der CF₂O-Gruppe verbunden. In der Druckschrift EP 1482018 A1 wird eine Substanz mit einem Cyclohexenring und einer CF₂O-Gruppe als synthetische Zwischenstufe offenbart, die nicht aufgereinigt oder charakterisiert wird. Die Verbindung besitzt eine endständige Ethylestergruppe für die weitere chemische Umsetzung.

In der Druckschrift EP 1053996 A1 wird eine Verbindung mit einer CF₂O-Gruppe zwischen zwei Cyclohexanringen offenbart. Die Druckschrift DE 10013681 A1 offenbart Fluorcyclohexenverbindungen. In der Druckschrift EP 1081123 A2 wird eine CF₂O-Gruppe zwischen einem Cyclohexenring und einem 2,3-Difluorbenzolring offenbart, wobei die Doppelbindung an das Sauerstoffatom gebunden ist.

Darüber hinaus wurden bereits verschiedene Verbindungen mit einer Difluormethylenoxy-Brücke und ohne einen Cyclohexenring als flüssigkristallines Material und dessen Herstellung beschrieben, wie z. B. in der Druckschrift EP 0786445 A1.

Der vorliegenden Erfindung lag die Aufgabe zugrunde, neue stabile flüssigkristalline oder mesogene Verbindungen aufzufinden, die als Komponente(n) flüssigkristalliner Medien geeignet sind. Insbesondere sollen die Verbindungen gleichzeitig eine vergleichsweise geringe Viskosität, sowie eine dielektrische Anisotropie im positiven Bereich besitzen. Für viele aktuelle Mischungskonzepte im Bereich der Flüssigkristalle ist es vorteilhaft, Verbindungen mit einer hohen dielektrischen Anisotropie Δε zu verwenden.

Im Hinblick auf die verschiedensten Einsatzbereiche derartiger Verbindungen mit hohem Δε war es wünschenswert, weitere Verbindungen, vorzugsweise mit hoher Nematogenität zur Verfügung zu haben, die auf die jeweiligen Anwendungen genau maßgeschneiderte Eigenschaften aufweisen.

Der Erfindung lag somit als eine Aufgabe zugrunde, neue stabile, flüssigkristalline oder mesogene Verbindungen aufzufinden, die als Komponente(n) flüssigkristalliner Medien, insbesondere für z. B. TN-, STN-, IPS- und TN-TFT-Displays, geeignet sind.

Eine weitere Aufgabe der vorliegenden Erfindung war es, flüssigkristalline oder mesogene Verbindungen bereitzustellen, die für sich oder in Mischungen eine hohe dielektrische Anisotropie Δε, einen hohen Klärpunkt sowie eine niedrige Rotationsviskosität γ₁ aufweisen. Darüber hinaus sollten die erfindungsgemäßen Verbindungen unter den in den Anwendungsgebieten vorherrrschenden Bedingungen thermisch und photochemisch stabil sein. Ferner sollten die erfindungsgemäßen Verbindungen möglichst eine breite nematische Phase aufweisen. Als Mesogene sollten sie eine breite nematische Phase in Mischungen mit flüssigkristallinen Cokomponenten ermöglichen sowie hervorragend mit nematischen Basismischungen, insbesondere bei tiefen Temperaturen, mischbar sein. Ebenso bevorzugt sind Substanzen mit einem niederigen Schmelzpunkt und einer geringen Schmelzenthalpie, da diese Größen wiederum Anzeichen für die oben genannten wünschenswerten Eigenschaften sind, wie z. B. eine hohe Löslichkeit und eine breite flüssigkristalline Phase etc..

Überraschenderweise wurde gefunden, dass die erfindungsgemäßen Cyclohexen-Derivate vorzüglich als Komponenten flüssigkristalliner Medien geeignet sind. Mit ihrer Hilfe lassen sich flüssigkristalline Medien, insbesondere geeignet für TN-TFT- und STN-Displays, aber auch für IPS-Systeme oder neuere Konzepte, die besonders hohe dielektrische Anisotropien benötigen, erhalten. Die erfindungsgemäßen Verbindungen sind hinreichend stabil und farblos. Auch zeichnen sie sich durch stark positive dielektrische Anisotropien Δε aus, aufgrund derer in der Anwendung in optischen Schaltelementen niedrigere Schwellenspannungen erforderlich sind. Sie besitzen einen besonders breiten nematischen Phasenbereich. Darüber hinaus weisen die erfindungsgemäßen Verbindungen einen hohen Klärpunkt sowie gleichzeitig niedere Werte für die Rotationsviskosität auf. Im Vergleich mit Stoffen aus dem Stand der Technik werden deutlich niedrigere Schmelzpunkte und Schmelzenthalpien beobachtet.

Mit der Bereitstellung der erfindungsgemäßen Cyclohexen-Derivate wird ganz allgemein die Palette der flüssigkristallinen Substanzen, die sich unter verschiedenen, anwendungstechnischen Gesichtspunkten zur Herstellung flüssigkristalliner Mischungen eignen, erheblich verbreitert.

Die erfindungsgemäßen Cyclohexen-Derivate besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind. Es können aber auch den erfindungsgemäßen Verbindungen flüssigkristalline Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Gegenstand der Erfindung sind somit Verbindungen der Formel I, 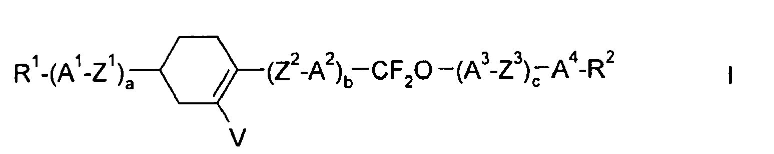 worin
- R¹: jeweils unabhängig voneinander H, F, Cl, Br, einen halogenierten oder unsubstituierten Alkylrest mit 1 bis 15 C-Atomen, wobei in diesen Resten auch eine oder mehrere CH₂-Gruppen jeweils unabhängig voneinander durch -C≡C-, -CH=CH- , -(CO)O-, -O(CO)-, -(CO)- oder -O-so ersetzt sein können, dass O-Atome nicht direkt miteinander verknüpft sind, wobei R¹ auch CN, SCN, NCS oder SF₅ bedeuten kann,
mit der Maßgabe, dass R¹ keine Estergruppe der Formel -(CO)O-C₂H₅, insbesondere nicht der Formel -(CO)O-Alkyl, ist.
- R²: einen Rest X der Bedeutung F, Cl, OCF₃, OCHF₂, OCHFCF₃, OCF₂CHFCF₃, CF₃, CN, SF₅ oder NCS,
- A¹ und A²: jeweils unabhängig voneinander, gleich oder verschieden:
a) trans-1,4-Cyclohexylen oder Cyclohexenylen, worin auch eine oder mehrere nicht benachbarte CH₂-Gruppen durch -O- und/oder -S- ersetzt sein können und worin H durch F substituiert sein kann,
b) 1,4-Phenylen, worin eine oder zwei CH-Gruppen durch N ersetzt sein können und worin auch ein oder mehrere H-Atome gegen Br, Cl, F, CN, Methyl, Methoxy oder eine ein- oder mehrfach fluorierte Methyl- oder Methoxygruppe ersetzt sein können,
oder
c) ein Rest aus der Gruppe 1,4-Bicyclo(2,2,2)-octylen, Piperidin-1,4-diyl, Cylcobut-1,3-diyl, Spiro[3.3]heptan-2,6-diyl, 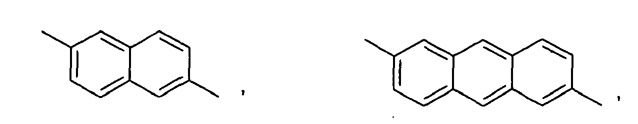 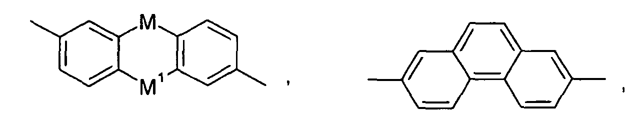 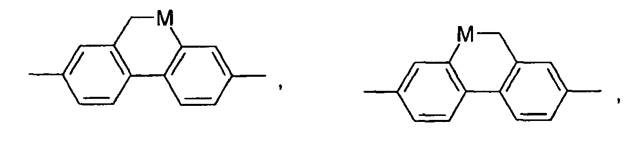 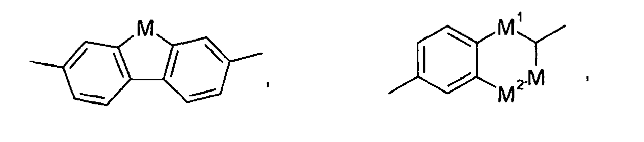 worin Wasserstoffatome ein oder mehrfach durch F, CN, SCN, SF₅, CH₂F, CHF₂, CF₃, OCH₂F, OCHF₂ oder OCF₃ substituiert sein können,
eine oder mehrere Doppelbindungen durch Einfachbindungen ersetzt sein können,
M, M¹ oder M² -O-, -S-, -CH₂-, -CHY- oder -CYY¹-, so dass benachbarte Gruppen nicht gleichzeitig -O- oder -S-bedeuten, und
Y und Y¹ Cl, F, CN, OCF₃ oder CF₃ bedeuten,
- A³ und A⁴: jeweils unabhängig voneinander, gleich oder verschieden, wie b) oder c) für A¹,
- V: H oder F,
- Z¹, Z² und Z³: jeweils unabhängig voneinander, gleich oder verschieden, eine Einfachbindung, -CH₂O-, -(CO)O-, -CF₂O-, -CH_{2C}H₂CF₂O-, -CF₂CF₂-, -CH₂CF₂-, -CH₂CH₂-, -(CH₂)₄-, -CH=CH-, -CH=CF-, -CF=CF- oder -C≡C-, wobei asymmetrische Brücken nach beiden Seiten orientiert sein können, und
- a: 0, 1 oder 2, bevorzugt 0 oder 1,
- b: 1 oder 2, bevorzugt 1, und
- c: 0, 1 oder 2, bevorzugt 0,
wobei a + b + c ≤ 4 ist,
bedeuten.

A¹⁻³ und Z¹⁻³ können unabhängig auch verschiedene Bedeutungen annehmen wenn sie für a, b oder c > 1 mehrmals auftreten.

Gegenstand der Erfindung ist weiterhin die Verwendung der Verbindungen der Formel I in flüssigkristallinen Medien.

Ebenfalls Gegenstand der vorliegenden Erfindung sind flüssigkristalline Medien mit mindestens zwei flüssigkristallinen Komponenten, welche mindestens ein Cyclohexen-Derivat der Formel I enthalten.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Die Verbindungen der Formel I sind in reinem Zustand farblos und bilden für sich oder in Mischungen flüssigkristalline Mesophasen in einem für die elektrooptische Verwendung günstig gelegenen Temperaturbereich. Mit den erfindungsgemäßen Verbindungen lassen sich breite nematischen Phasenbereiche erzielen. In flüssigkristallinen Mischungen unterdrücken die erfindungsgemäßen Substanzen die smektischen Phasen und führen zu einer deutlichen Verbesserung der Tieftemperatur-Lagerstabilität. Bevorzugt sind Verbindungen der Formel I, worin a 0 oder 1, insbesondere a = 1, ist.

Z¹ und/oder Z³ bedeuten bevorzugt eine Einfachbindung, -CF₂O-, -OCF₂-, -C₂F₄-, -CH₂O- -OCH₂- oder -(CO)O-, insbesondere eine Einfachbindung. Z² bedeutet bevorzugt -CH₂CH₂-, -CH=CH-, -C=C- oder eine Einfachbindung, insbesondere eine Einfachbindung.

Für den Fall, dass Z² eine Einfachbindung ist, bedeutet A² bevorzugt einen ungesättigten oder aromatischen Ring aus den Gruppen b) oder c) nach der Definition der Formel I. In diesem Fall steht die Doppelbindung des Cyclohexens mit dem benachbarten ungesättigten Ring A² in Konjugation.

A¹, A², A³ und A⁴ bedeuten bevorzugt 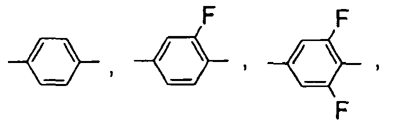 A¹, A² auch 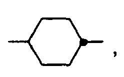 und ferner, 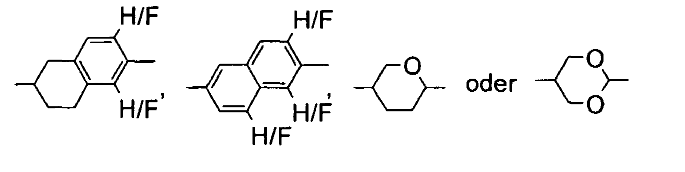 A² bedeutet bevorzugt 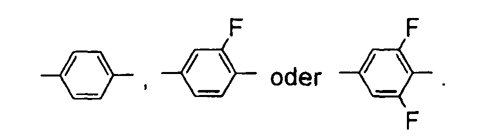 A⁴ bedeutet bevorzugt 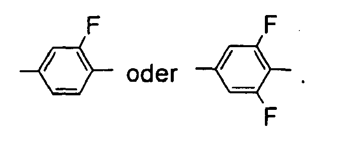 R¹ bedeutet bevorzugt Alkyl, Alkoxy, Alkenyl oder Alkenyloxy mit bis zu 8 Kohlenstoffatomen. R¹ bedeutet besonders bevorzugt geradkettiges Alkyl oder Alkenyl.
Bevorzugt bedeutet R² X, wobei
X F, Cl, OCF₃, OCHF₂, OCHFCF₃, OCF₂CHFCF₃, CF₃, CN, SF₅, NCS, insbesondere F, Cl, CN oder OCF₃ und ganz besonders F.
Vorzugsweise bedeuten R¹ und R² nicht gleichzeitig H.

Besonders bevorzugt sind Verbindungen der Formeln IA 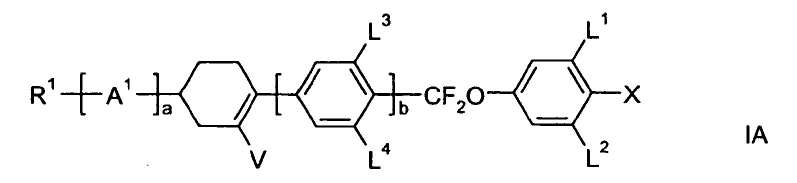 worin
R¹, A¹, X, a, b und V die oben für Formel I angegebenen Bedeutungen haben, sowie
L¹, L², L³ und L⁴
H oder F
bedeuten.

Bevorzugt sind Verbindungen der Formel IA worin L¹ ein Fluor bedeutet b bedeutet bevorzugt 1. V ist bevorzugt H. L³ ist bevorzugt F.

Besonders bevorzugte Verbindungen der Formel I sind die Verbindungen der Formeln I1 bis I5, 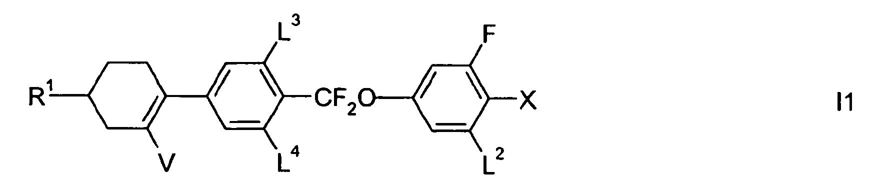 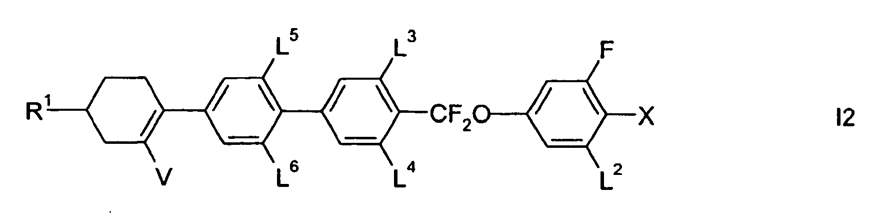 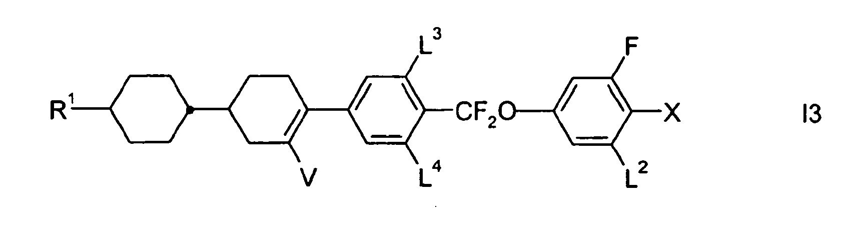 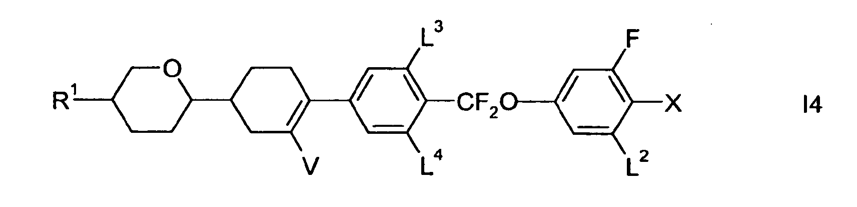 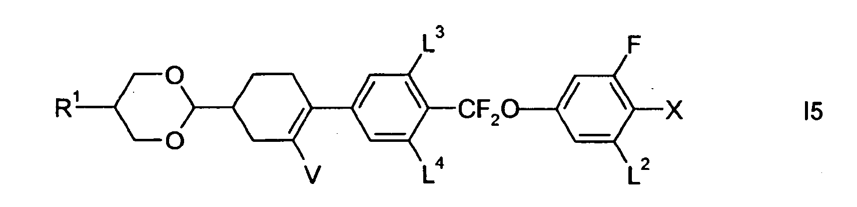 worin R¹, V und X die oben angegebenen Bedeutungen haben. L², L³, L⁴, L⁵ und L⁶ bedeuten unabhängig voneinander H oder F.

Bei Verbindungen, die in Diastereomeren auftreten können, sind sowohl die Reinsubstanzen als auch jedes Mischungsverhältnis der Isomeren umfasst und jeweils als geeignete Mischungskomponente anzusehen.

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart) beschrieben sind und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Verbindungen der Formel I können vorteilhafterweise wie an den folgenden beispielhaften Synthesen ersichtlich hergestellt werden (Schema 1 und 2):

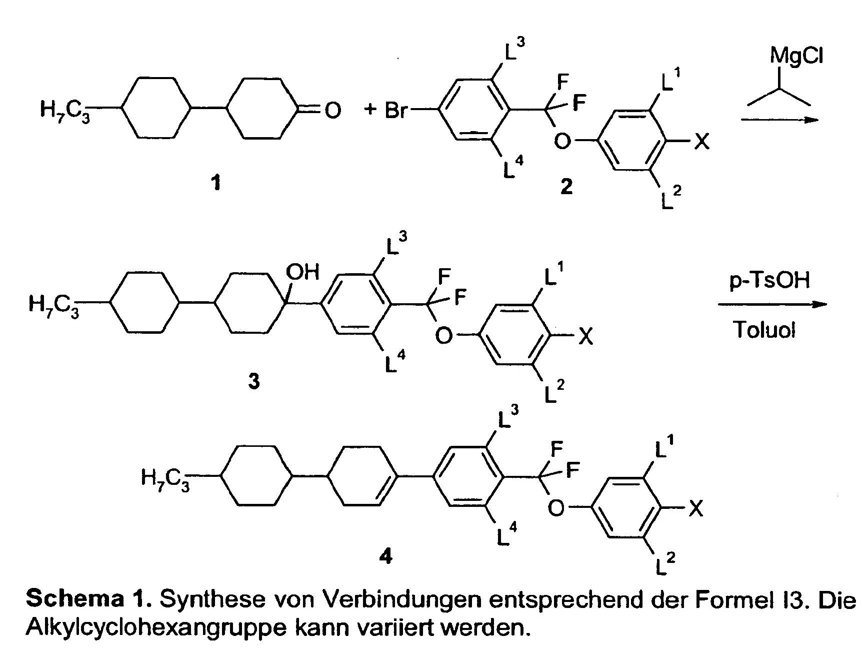

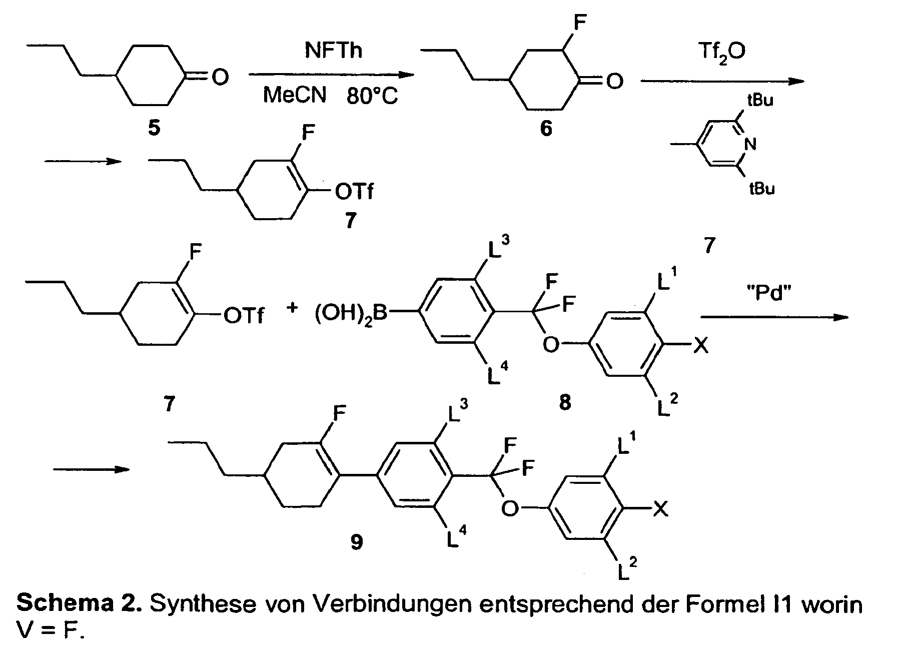

Die Molekülgruppen haben dabei die folgenden Bedeutungen:

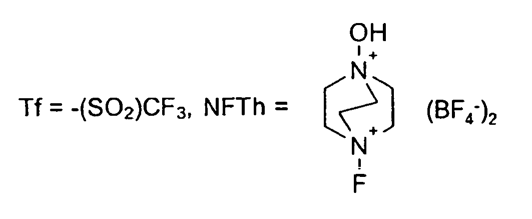

Die unbeteiligten Gruppen der Formeln in Schema 1 und 2 lassen sich variieren, so weit es die Verbindungen der Formel I nah legen. Entsprechende Ausgangsprodukte lassen sich in der Regel vom Fachmann ohne weiteres herstellen. Verallgemeinert lassen sich so die Verbindungen der Formeln I oder IA herstellen.

Die Erfindung hat daher auch zwei Verfahrensvarianten zur Herstellung von Verbindungen der Formel I zum Gegenstand:
a) Ein Verfahren zur Herstellung von Verbindungen der Formel I worin V Wasserstoff bedeutet umfassend einen Verfahrensschritt, der dadurch gekennzeichnet ist, dass ein Cyclohexanketon der Formel 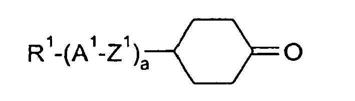 worin R¹, A¹, Z¹ und a wie in Anspruch 1 definiert sind,
   mit einer Magnesium-organischen Verbindung der Formel

   HalMg-(Z²-A²)_{b}CF₂O-(A³-Z³)_{c}-A⁴-R²

   worin Z², Z³, A², A³, A⁴, b, c und R² wie in Anspruch 1 definiert sind und
   Hal Cl oder Br bedeutet,
   zur Reaktion gebracht wird. Bevorzugt wird der nach der Aufarbeitung entstandene Alkohol zu einer Cyclohexenverbindung der Formel I eliminiert. Bevorzugt erreicht man das durch Zugabe einer katalytischen Menge Säure, insbesondere von p-Toluolsulfonsäure (p-TsOH).
b) Ein Verfahren zur Herstellung von Verbindungen der Formel I nach Anspruch 1, worin V Wasserstoff oder Fluor bedeutet umfassend einen Verfahrensschritt, der dadurch gekennzeichnet ist, dass ein Cyclohexen der Formel 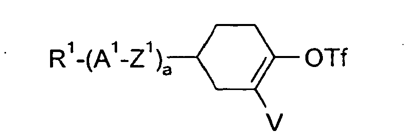 worin R¹, A¹, Z¹, V und a wie in Anspruch 1 definiert sind, mit einer Boronsäure oder einem offenkettigen oder cyclischen Boronsäureester der Formeln

   (OH)₂B-(Z²-A²)_{b}CF₂O-(A³-Z³)_{c}-A⁴-R²

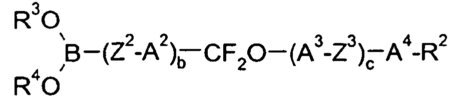

   worin Z², Z³, A², A³, A⁴, b, c und R² wie in Anspruch 1 definiert sind, und
   R³, R⁴ Alkyl mit 1-12 C-Atomen oder R³+R⁴ zusammen auch ein Alkylen, insbesondere der Formeln -CH₂-(CH₂)p-CH₂- und -C(CH₃)₂C(CH₃)₂-,
   oder 1,2-Phenylen bedeuten,
   wobei R¹; R² und R¹+R² auch substituiert sein können und
   wobei p 0 oder 1 ist,
   in Gegenwart eines Übergangsmetallkatalysators, bevorzugt eines Palladiumkomplexes, zur Reaktion gebracht wird. Bei den Komplexen handelt es sich bevorzugt um Bis(triphenylphosphin)palladium(II)-chlorid.

Erfindungsgemäße Verbindungen, worin A² ein optional substituiertes 1,4-Phenylen bedeutet und b den Wert 2 besitzt werden vorteilhafterweise analog gemäß Schema 3 hergestellt.

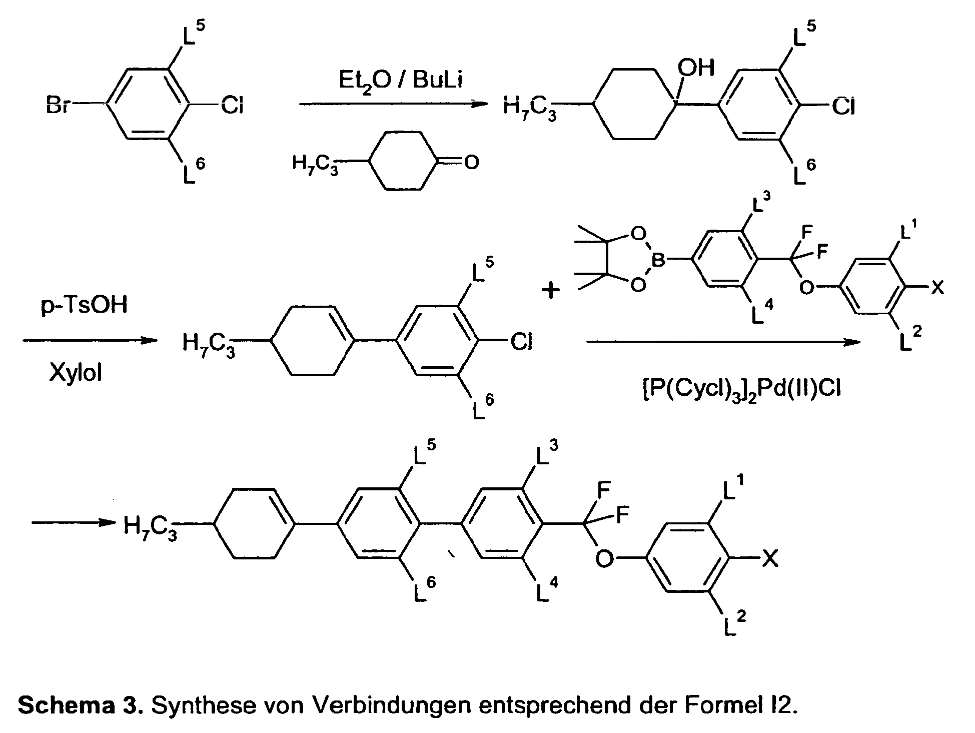

Weitere bevorzugte Verfahrensvarianten lassen sich den Beispielen entnehmen.

Gegenstand der Erfindung sind auch flüssigkristalline Medien enthaltend eine oder mehrere der erfindungsgemäßen Verbindungen der Formel I. Die flüssigkristallinen Medien enthalten wenigstens zwei Komponenten. Man erhält sie vorzugsweise indem man die Komponenten miteinander vermischt. Ein erfindungsgemäßes Verfahren zur Herstellung eines flüssigkristallinen Mediums ist daher dadurch gekennzeichnet, dass man mindestens eine Verbindung der Formel I mit mindestens einer weiteren mesogenen Verbindung vermischt und gegebenenfalls Additive zugibt. Die erzielbaren Kombinationen aus Klärpunkt, Viskosität bei tiefer Temperatur, thermischer und UV-Stabilität und dielektrischer Anisotropie übertreffen bei weitem bisherige Materialien aus dem Stand der Technik.

Die erfindungsgemäßen, flüssigkristallinen Medien enthalten vorzugsweise neben einer oder mehreren erfindungsgemäßen Verbindungen als weitere Bestandteile 2 bis 40, besonders bevorzugt 4 bis 30 Komponenten. Insbesondere enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexancarbonsäure-phenyl- oder cyclohexylester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexane, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylethane, 1-Phenyl-2-cyclohexyl-phenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile der erfindungsgemäßen Medien in Frage kommenden Verbindungen lassen sich durch die Formeln 1, 2, 3, 4 und 5 charakterisieren:

R'-L-E-R" 1

R'-L-COO-E-R" 2

R'-L-CF₂O-E-R" 3

R'-L-CH₂CH₂-E-R" 4

R'-L-C≡C-E-R" 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -Py-, -G-Phe- und -G-Cyc- sowie deren Spiegelbildern gebildeten Gruppe, wobei Phe unsubstituiertes oder durch Fluor substituiertes 1,4-Phenylen, Cyc trans-1,4-Cyclohexylen, Pyr Pyrimidin-2,5-diyl oder Pyridin-2,5-diyl, Dio 1,3-Dioxan-2,5-diyl, Py Tetrahydropyran-2,5-diyl- und G 2-(trans-1,4-Cyclohexyl)-ethyl bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe, Py und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und/oder R" bedeuten jeweils unabhängig voneinander Alkyl, Alkenyl, Alkoxy, Alkoxyalkyl, Alkenyloxy oder Alkanoyloxy mit bis zu 8 C-Atomen, -F, -Cl, -CN, -NCS oder -(O)ᵢCH₃₋ₖFₖ, wobei i 0 oder 1 und k 1, 2 oder 3 ist.

R' und R" bedeuten in einer kleineren Untergruppe der Verbindungen der Formeln 1, 2, 3, 4 und 5 jeweils unabhängig voneinander Alkyl, Alkenyl, Alkoxy, Alkoxyalkyl, Alkenyloxy oder Alkanoyloxy mit bis zu 8 C-Atomen. Im folgenden wird diese kleinere Untergruppe Gruppe A genannt und die Verbindungen werden mit den Teilformeln 1a, 2a, 3a, 4a und 5a bezeichnet. Bei den meisten dieser Verbindungen sind R' und R" voneinander verschieden, wobei einer dieser Reste meist Alkyl, Alkenyl, Alkoxy oder Alkoxyalkyl ist.

In einer anderen als Gruppe B bezeichneten kleineren Untergruppe der Verbindungen der Formeln 1, 2, 3, 4 und 5 bedeutet R" -F, -Cl, -NCS oder -(O)ᵢCH₃₋ₖFₖ, wobei i 0 oder 1 und k 1, 2 oder 3 ist. Die Verbindungen, in denen R" diese Bedeutung hat, werden mit den Teilformeln 1 b, 2b, 3b, 4b und 5b bezeichnet. Besonders bevorzugt sind solche Verbindungen der Teilformeln 1 b, 2b, 3b, 4b und 5b, in denen R" die Bedeutung -F, -Cl, -NCS, -CF₃, -OCHF₂ oder -OCF₃ hat.

In den Verbindungen der Teilformeln 1 b, 2b, 3b, 4b und 5b hat R' die bei den Verbindungen der Teilformeln 1a bis 5a angegebenen Bedeutungen und ist vorzugsweise Alkyl, Alkenyl, Alkoxy oder Alkoxyalkyl.

In einer weiteren kleineren Untergruppe der Verbindungen der Formeln 1, 2, 3, 4 und 5 bedeutet R" -CN. Diese Untergruppe wird im folgenden als Gruppe C bezeichnet und die Verbindungen dieser Untergruppe werden entsprechend mit Teilformeln 1 c, 2c, 3c, 4c und 5c beschrieben. In den Verbindungen der Teilformeln 1c, 2c, 3c, 4c und 5c hat R' die bei den Verbindungen der Teilformeln 1a bis 5a angegebenen Bedeutungen und ist vorzugsweise Alkyl, Alkoxy oder Alkenyl.

Neben den bevorzugten Verbindungen der Gruppen A, B und C sind auch andere Verbindungen der Formeln 1, 2, 3, 4 und 5 mit anderen Varianten der vorgesehenen Substituenten gebräuchlich. All diese Substanzen sind nach literaturbekannten Methoden oder in Analogie dazu erhältlich.

Die erfindungsgemäßen Medien enthalten neben erfindungsgemäßen Verbindungen der Formel I vorzugsweise eine oder mehrere Verbindungen, welche ausgewählt werden aus den Gruppen A, B und/oder C. Die Massenanteile der Verbindungen aus diesen Gruppen in den erfindungsgemäßen Medien sind vorzugsweise:
- Gruppe A:: 0 bis 90 %, vorzugsweise 20 bis 90 %, besonders bevorzugt 30 bis 90 %;
- Gruppe B:: 0 bis 80 %, vorzugsweise 10 bis 80 %, besonders bevorzugt 10 bis 65 %;
- Gruppe C:: 0 bis 80 %, vorzugsweise 0 bis 80 %, besonders bevorzugt 0 bis 50 %;
wobei die Summe der Massenanteile der in den jeweiligen erfindungsgemäßen Medien enthaltenen Verbindungen aus den Gruppen A, B und/oder C vorzugsweise 5 bis 90 % und besonders bevorzugt 10 bis 90 % beträgt.

Die erfindungsgemäßen Medien enthalten vorzugsweise 1 bis 40 %, besonders bevorzugt 5 bis 30 %, der erfindungsgemäßen Verbindungen.

Die Herstellung der erfindungsgemäßen Flüssigkristallmischungen erfolgt in an sich üblicher Weise. In der Regel wird die gewünschte Menge der in geringerer Menge verwendeten Komponenten in der den Hauptbestandteil ausmachenden Komponenten gelöst, vorzugsweise bei erhöhter Temperatur. Es ist auch möglich, Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation. Weiterhin ist es möglich, die Mischungen auf andere herkömmliche Arten, z. B. durch Verwendung von Vormischungen, z.B. Homologen-Mischungen oder unter Verwendung von sogenannten "Multi-Bottle"-Systemen herzustellen.

Die Dielektrika können auch weitere, dem Fachmann bekannte und in der Literatur beschriebene Zusätze enthalten. Beispielsweise können 0 bis 15 %, vorzugsweise 0 bis 10 %, pleochroitische Farbstoffe, chirale Dotierstoffe, Stabilisatoren oder Nanopartikel zugesetzt werden. Die einzelnen, zugesetzten Verbindungen werden in Konzentrationen von 0,01 bis 6 %, vorzugsweise von 0,1 bis 3 %, eingesetzt. Dabei werden jedoch die Konzentrationsangaben der übrigen Bestandteile der Flüssigkristallmischungen also der flüssigkristallinen oder mesogenen Verbindungen, ohne Berücksichtigung der Konzentration dieser Zusatzstoffe angegeben. Die erfindungsgemäßen Flüssigkristallmischungen ermöglichen eine bedeutende Erweiterung des zur Verfügung stehenden Parameterraumes. Gegenstand der Erfindung sind auch elektrooptische Anzeigen (insbesondere TFT-Anzeigen mit zwei planparallelen Trägerplatten, die mit einer Umrandung eine Zelle bilden, integrierten nicht-linearen Elementen zur Schaltung einzelner Bildpunkte auf den Trägerplatten und einer in der Zelle befindlichen nematischen Flüssigkristallmischung mit positiver dielektrischer Anisotropie und hohem spezifischem Widerstand), die derartige Medien enthalten sowie die Verwendung dieser Medien für elektrooptische Zwecke.

Der Ausdruck "Alkyl" umfasst geradkettige und verzweigte Alkylgruppen mit 1-9 Kohlenstoffatomen, insbesondere die geradkettigen Gruppen Methyl, Ethyl, Propyl, Butyl, Pentyl, Hexyl und Heptyl. Gruppen mit 2-5 Kohlenstoffatomen sind im allgemeinen bevorzugt.

Der Ausdruck "Alkenyl" umfasst geradkettige und verzweigte Alkenylgruppen mit bis zu 9 Kohlenstoffatomen, insbesondere die geradkettigen Gruppen. Besonders bevorzugte Alkenylgruppen sind C₂-C₇-1E-Alkenyl, C₄-C₇-3E-Alkenyl, C₅-C₇-4-Alkenyl, C₆-C₇-5-Alkenyl und C₇-6-Alkenyl, insbesondere C₂-C₇-1 E-Alkenyl, C₄-C₇-3E-Alkenyl und C₅-C₇-4-Alkenyl. Beispiele bevorzugter Alkenylgruppen sind Vinyl, 1 E-Propenyl, 1 E-Butenyl, 1 E-Pentenyl, 1 E-Hexenyl, 1 E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im allgemeinen bevorzugt.

Der Ausdruck "halogenierter Alkylrest" umfasst vorzugsweise ein- oder mehrfach fluorierte und/oder chlorierte Reste. Perhalogenierte Reste sind eingeschlossen. Besonders bevorzugt sind fluorierte Alkylreste, insbesondere CF₃, CH₂CF₃, CH₂CHF₂, CHF₂, CH₂F, CHFCF₃ und CF₂CHFCF₃.

Die Gesamtmenge an Verbindungen der Formeln I in den erfindungsgemäßen Gemischen ist nicht kritisch. Die Gemische können daher eine oder mehrere weitere Komponenten enthalten zwecks Optimierung verschiedener Eigenschaften. Der beobachtete Effekt auf die Ansprechzeiten und die Schwellenspannung ist jedoch in der Regel umso größer je höher die Gesamtkonzentration an Verbindungen der Formeln I bis XV ist.

Der Aufbau der erfindungsgemäßen Matrix-Anzeige aus Polarisatoren, Elektrodengrundplatten und Elektroden mit Oberflächenbehandlung entspricht der für derartige Anzeigen üblichen Bauweise. Dabei ist der Begriff der üblichen Bauweise hier weit gefasst und umfasst auch alle Abwandlungen und Modifikationen der Matrix-Anzeige, insbesondere auch Matrix-Anzeigeelemente auf Basis von poly-Si TFT.

Ein wesentlicher Unterschied der erfindungsgemäßen Anzeigen zu den bisher üblichen auf der Basis der verdrillten nematischen Zelle besteht jedoch in der Wahl der Flüssigkristallparameter der Flüssigkristallschicht.

Die folgenden Beispiele sollen die Erfindung erläutern, ohne sie zu begrenzen. Vor- und nachstehend bedeuten Prozentangaben Gewichtsprozent. Alle Temperaturen sind in Grad Celsius angegeben. Ferner bedeuten K = kristalliner Zustand, N = nematische Phase, S = smektische Phase und I = isotrope Phase. Die Angaben zwischen diesen Symbolen stellen die Übergangstemperaturen dar. Δn bedeutet optische Anisotropie (589 nm, 20 °C), Δε die dielektrische Anisotropie (1kHz, 20 °C) und γ₁ die Rotationsviskosität (in der Einheit mPa·s).

Die Bestimmung physikalischer, physikochemischer beziehungsweise elektrooptischer Parameter erfolgt nach allgemein bekannten Verfahren, wie sie unter anderem beschrieben sind in der Broschüre "Merck Liquid Crystals - Licristal® - Physical Properties of Liquid Crystals - Description of the Measurements Methods", 1998, Merck KGaA, Darmstadt.

Die dielektrische Anisotropie Δε der einzelnen Substanzen wird bei 20 °C und 1 kHz bestimmt. Dazu werden 5-10 Gew.% der zu untersuchenden Substanz in der dielektrisch positiven Mischung ZLI-4792 (Merck KGaA) gelöst gemessen und der Messwert auf eine Konzentration von 100 % extrapoliert. Die optische Anisotropie Δn wird bei 20°C und einer Wellenlänge von 589,3 nm bestimmt, die Rotationsviskosität γ₁ bei 20°C, beide ebenfalls durch lineare Extrapolation.

Folgende Abkürzungen werden verwendet:
- p-TsOH: p-Toluolsulfonsäure
- THF: Tetrahydrofuran
- MTB-Ether: Methyl-*t*-butylether

### Beispiel 1

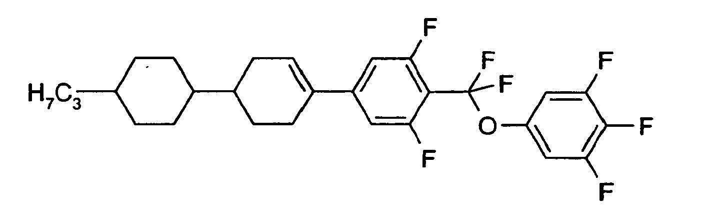

### Schritt 1.1

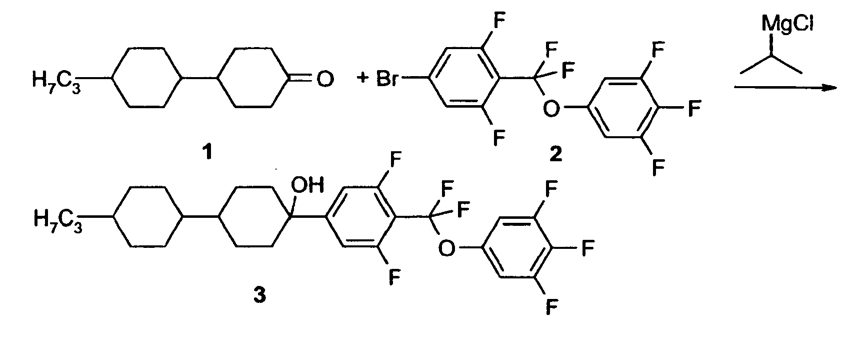

96 ml (190 mmol) einer 2 M Lösung von Isopropylmagnesiumchlorid in THF werden unter Stickstoff bei 20°C mit einer Lösung von 62,1 g (160 mmol) des Bromids **2** in 300 ml THF versetzt. Nach 1 h werden 29,0 g (190 mmol) des Ketons **1** gelöst in 200 ml THF bei ebenfalls 20 °C zugegeben. Nach einer weiteren Stunde wird der Ansatz mit 250 ml Wasser hydrolysiert, mit 1 N Salzsäure angesäuert und mit 500 ml Heptan verdünnt. Die wässerige Phase wird mit MTB-Ether extrahiert. Die vereinigten organischen Phasen werden mit ges. Natriumhydrogencarbonatlsg. gewaschen, über Natriumsulfat getrocknet und eingeengt. Die Substanz **3** wird ohne weitere Reinigung in der Folgestufe eingesetzt.

### Schritt 1.2

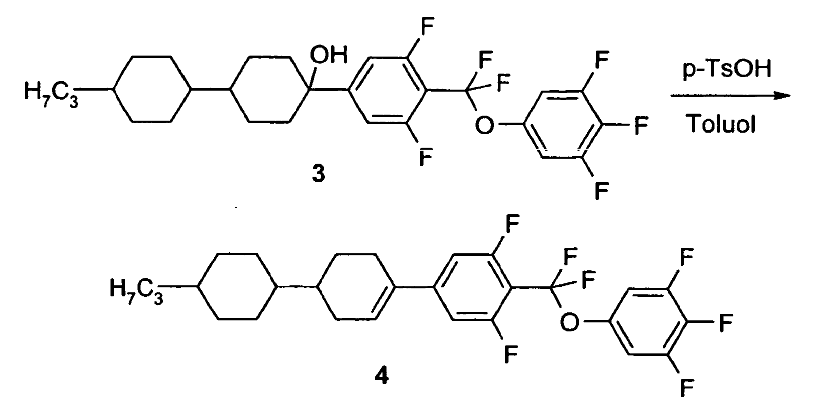

68,1 g (130 mmol) des Alkohols **1** werden in 400 ml Toluol gelöst und mit 1,1 g (10 mmol) p-Toluolsulfonsäure-Monohydrat versetzt und 2 h am Wasserabscheider erhitzt. Anschließend wird das Lösungsmittel entfernt und der erhaltene Rückstand mit Toluol über Kieselgel gegeben. Kristallisation aus Ethanol und n-Heptan.
K 63 N 155 I
Δε 21
Δn 0,132

Analog werden die folgenden Verbindungen der Formel 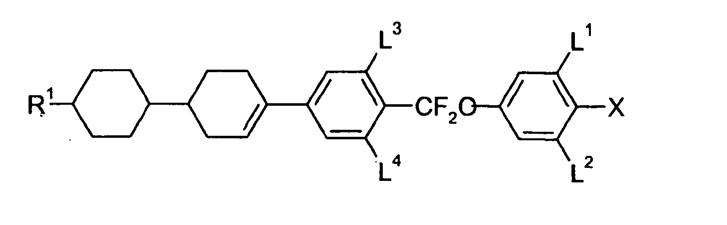 hergestellt:

| R¹ | X | L¹ | L² | L³ | L⁴ | Werte |
|---|---|---|---|---|---|---|
| H | F | H | H | H | H | |
| CH₃ | F | H | H | H | H | |
| C₂H₅ | F | H | H | H | H | |
| C₃H₇ | F | H | H | H | H | |
| n-C₄H₉ | F | H | H | H | H | |
| n-C₅H₁₁ | F | H | H | H | H | |
| n-C₆H1₃ | F | H | H | H | H | |
| H | F | F | H | H | H | |
| CH₃ | F | F | H | H | H | |
| C₂H₅ | F | F | H | H | H | |
| n-C₃H₇ | F | F | H | H | H | K 56 Sm 81 SmB 105 N 207 I; Δε 12; Δn 0,155 |
| n-C₄H₉ | F | F | H | H | H | |
| n-C₅H₁₁ | F | F | H | H | H | |
| n-C₆H₁₃ | F | F | H | H | H | |
| H | F | F | F | H | H | |
| CH₃ | F | F | F | H | H | |
| C₂H₅ | F | F | F | H | H | |
| n-C₃H₇ | F | F | F | H | H | K 81 N 183 I; Δε 16; Δn 0,148 |
| n-C₄H₉ | F | F | F | H | H | |
| n-C₅H₁₁ | F | F | F | H | H | |
| n-C₆H₁₃ | F | F | F | H | H | |
| H | OCF₃ | H | H | H | H | |
| CH₃ | OCF₃ | H | H | H | H | |
| C₂H₅ | OCF₃ | H | H | H | H | |
| n-C₃H₇ | OCF₃ | H | H | H | H | |
| n-C₄H₉ | OCF₃ | H | H | H | H | |
| n-C₅H₁₁ | OCF₃ | H | H | H | H | |
| n-C₆H₁₃ | OCF₃ | H | H | H | H | |
| H | OCF₃ | F | H | H | H | |
| CH₃ | OCF₃ | F | H | H | H | |
| C₂H₅ | OCF₃ | F | H | H | H | |
| n-C₃H₇ | OCF₃ | F | H | H | H | K 59 Sm 117 SmB 127 SmC 129 N 209 I; Δε 14; Δn 0,150 |
| n-C₄H₉ | OCF₃ | F | H | H | H | |
| n-C₅H₁₁ | OCF₃ | F | H | H | H | |
| n-C₆H₁₃ | OCF₃ | F | H | H | H | |
| H | OCF₃ | F | F | H | H | |
| CH₃ | OCF₃ | F | F | H | H | |
| C₂H₅ | OCF₃ | F | F | H | H | |
| n-C₃H₇ | OCF₃ | F | F | H | H | K 58 SmB 76 N 191 I; Δε 16; Δn 0,142 |
| n-C₄H₉ | OCF₃ | F | F | H | H | |
| n-C₅H₁₁ | OCF₃ | F | F | H | H | |
| n-C₆H₁₃ | OCF₃ | F | F | H | H | |
| H | CN | H | H | H | H | |
| CH₃ | CN | H | H | H | H | |
| C₂H₅ | CN | H | H | H | H | |
| n-C₃H₇ | CN | H | H | H | H | |
| n-C₄H₉ | CN | H | H | H | H | |
| n-C₅H₁₁ | CN | H | H | H | H | |
| n-C₆H₁₃ | CN | H | H | H | H | |
| H | CN | F | H | H | H | |
| CH₃ | CN | F | H | H | H | |
| C₂H₅ | CN | F | H | H | H | |
| n-C₃H₇ | CN | F | H | H | H | |
| n-C₄H₉ | CN | F | H | H | H | |
| n-C₅H₁₁ | CN | F | H | H | H | |
| n-C₆H₁₃ | CN | F | H | H | H | |
| H | CN | F | F | H | H | |
| CH₃ | CN | F | F | H | H | |
| C₂H₅ | CN | F | F | H | H | |
| n-C₃H₇ | CN | F | F | H | H | |
| n-C₄H₉ | CN | F | F | H | H | |
| n-C₅H₁₁ | CN | F | F | H | H | |
| n-C₆H₁₃ | CN | F | F | H | H | |
| H | F | H | H | F | H | |
| CH₃ | F | H | H | F | H | |
| C₂H₅ | F | H | H | F | H | |
| C₃H₇ | F | H | H | F | H | |
| n-C₄H₉ | F | H | H | F | H | |
| n-C₅H₁₁ | F | H | H | F | H | |
| n-C₆H₁₃ | F | H | H | F | H | |
| H | F | F | H | F | H | |
| CH₃ | F | F | H | F | H | |
| C₂H₅ | F | F | H | F | H | |
| n-C₃H₇ | F | F | H | F | H | |
| n-C₄H₉ | F | F | H | F | H | |
| n-C₅H₁₁ | F | F | H | F | H | |
| n-C₆H₁₃ | F | F | H | F | H | |
| H | F | F | F | F | H | |
| CH₃ | F | F | F | F | H | |
| C₂Hs | F | F | F | F | H | |
| n-C₃H₇ | F | F | F | F | H | |
| n-C₄H₉ | F | F | F | F | H | |
| n-C₅H₁₁ | F | F | F | F | H | |
| n-C₆H₁₃ | F | F | F | F | H | |
| H | OCF₃ | H | H | F | H | |
| CH₃ | OCF₃ | H | H | F | H | |
| C₂H₅ | OCF₃ | H | H | F | H | |
| n-C₃H₇ | OCF₃ | H | H | F | H | |
| n-C₄H₉ | OCF₃ | H | H | F | H | |
| n-C₅H₁₁ | OCF₃ | H | H | F | H | |
| n-C₆H₁₃ | OCF₃ | H | H | F | H | |
| H | OCF₃ | F | H | F | H | |
| CH₃ | OCF₃ | F | H | F | H | |
| C₂H₅ | OCF₃ | F | H | F | H | |
| n-C₃H₇ | OCF₃ | F | H | F | H | |
| n-C₄H₉ | OCF₃ | F | H | F | H | |
| n-C₅H₁₁ | OCF₃ | F | H | F | H | |
| n-C₆H₁₃ | OCF₃ | F | H | F | H | |
| H | OCF₃ | F | F | F | H | |
| CH₃ | OCF₃ | F | F | F | H | |
| C₂H₅ | OCF₃ | F | F | F | H | |
| n-C₃H₇ | OCF₃ | F | F | F | H | |
| n-C₄H₉ | OCF₃ | F | F | F | H | |
| n-C₅H₁₁ | OCF₃ | F | F | F | H | |
| n-C₆H₁₃ | OCF₃ | F | F | F | H | |
| H | CN | H | H | F | H | |
| CH₃ | CN | H | H | F | H | |
| C₂H₅ | CN | H | H | F | H | |
| n-C₃H₇ | CN | H | H | F | H | |
| n-C₄H₉ | CN | H | H | F | H | |
| n-C₅H₁₁ | CN | H | H | F | H | |
| n-C₆H₁₃ | CN | H | H | F | H | |
| H | CN | F | H | F | H | |
| CH₃ | CN | F | H | F | H | |
| C₂H₅ | CN | F | H | F | H | |
| n-C₃H₇ | CN | F | H | F | H | |
| n-C₄H₉ | CN | F | H | F | H | |
| n-C₅H₁₁ | CN | F | H | F | H | |
| n-C₆H₁₃ | CN | F | H | F | H | |
| H | CN | F | F | F | H | |
| CH₃ | CN | F | F | F | H | |
| C₂H₅ | CN | F | F | F | H | |
| n-C₃H₇ | CN | F | F | F | H | |
| n-C₄H₉ | CN | F | F | F | H | |
| n-C₅H₁₁ | CN | F | F | F | H | |
| n-C₆H₁₃ | CN | F | F | F | H | |
| H | F | H | H | F | F | |
| CH₃ | F | H | H | F | F | |
| C₂H₅ | F | H | H | F | F | |
| n-C₄H₉ | F | H | H | F | F | |
| n-C₅H₁₁ | F | H | H | F | F | |
| n-C₆H₁₃ | F | H | H | F | F | |
| H | F | F | H | F | F | |
| CH₃ | F | F | H | F | F | |
| C₂H₅ | F | F | H | F | F | |
| n-C₃H₇ | F | F | H | F | F | |
| n-C₄H₉ | F | F | H | F | F | |
| n-C₅H₁₁ | F | F | H | F | F | |
| n-C₆H₁₃ | F | F | H | F | F | |
| H | F | F | F | F | F | |
| CH₃ | F | F | F | F | F | |
| -CH=CH₂ | F | F | F | F | F | |
| C₂H₅ | F | F | F | F | F | K 57 N 131 I; Δε 22; Δn 0,124; γ₁ 367 mPa·s |
| n-C₃H₇ | F | F | F | F | F | vgl. Beispiel 1 |
| n-C₄H₉ | F | F | F | F | F | K 65 N 150 I; Δε 21; Δn 0,124 |
| n-C₅H₁₁ | F | F | F | F | F | K 71 N 156 I; Δε 20; Δn 0,133 |
| n-C₆H₁₃ | F | F | F | F | F | |
| n-C₇H₁₅ | F | F | F | F | F | |
| H | OCF₃ | H | H | F | F | |
| CH₃ | OCF₃ | H | H | F | F | |
| C₂H₅ | OCF₃ | H | H | F | F | |
| n-C₃H₇ | OCF₃ | H | H | F | F | |
| n-C₄H₉ | OCF₃ | H | H | F | F | |
| n-C₅H₁₁ | OCF₃ | H | H | F | F | |
| n-C₆H₁₃ | OCF₃ | H | H | F | F | |
| H | OCF₃ | F | H | F | F | |
| CH₃ | OCF₃ | F | H | F | F | |
| C₂H₅ | OCF₃ | F | H | F | F | |
| n-C₃H₇ | OCF₃ | F | H | F | F | |
| n-C₄H₉ | OCF₃ | F | H | F | F | |
| n-C₅H₁₁ | OCF₃ | F | H | F | F | |
| n-C₆H₁₃ | OCF₃ | F | H | F | F | |
| H | OCF₃ | F | F | F | F | |
| CH₃ | OCF₃ | F | F | F | F | |
| C₂H₅ | OCF₃ | F | F | F | F | |
| n-C₃H₇ | OCF₃ | F | F | F | F | |
| n-C₄H₉ | OCF₃ | F | F | F | F | |
| n-C₅H₁₁ | OCF₃ | F | F | F | F | |
| n-C₆H₁₃ | OCF₃ | F | F | F | F | |
| H | CN | H | H | F | F | |
| CH₃ | CN | H | H | F | F | |
| C₂H₅ | CN | H | H | F | F | |
| n-C₃H₇ | CN | H | H | F | F | |
| n-C₄H₉ | CN | H | H | F | F | |
| n-C₅H₁₁ | CN | H | H | F | F | |
| n-C₆H₁₃ | CN | H | H | F | F | |
| H | CN | F | H | F | F | |
| CH₃ | CN | F | H | F | F | |
| C₂H₅ | CN | F | H | F | F | |
| n-C₃H₇ | CN | F | H | F | F | |
| n-C₄H₉ | CN | F | H | F | F | |
| n-C₅H₁₁ | CN | F | H | F | F | |
| n-C₆H₁₃ | CN | F | H | F | F | |
| H | CN | F | F | F | F | |
| CH₃ | CN | F | F | F | F | |
| C₂H₅ | CN | F | F | F | F | |
| n-C₃H₇ | CN | F | F | F | F | |
| n-C₄H₉ | CN | F | F | F | F | |
| n-C₅H₁₁ | CN | F | F | F | F | |
| n-C₆H₁₃ | CN | F | F | F | F | |
| n-C₇H₁₅ | CN | F | F | F | F | |

### Beispiel 2

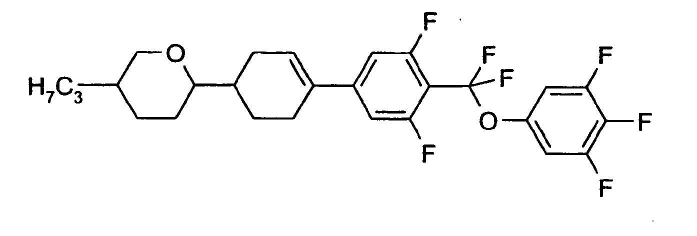

### Schritt 2.1

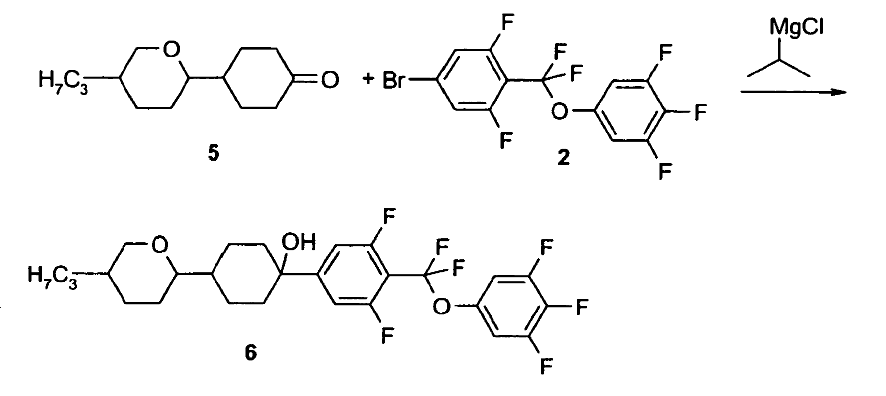

Analog Beispiel 1, Schritt 1.1 wird das Cyclohexanketon 5 mit dem Bromid 2, das zuvor metalliert wird, umgesetzt. Die Substanz 6 wird ohne weitere Reinigung in der Folgestufe eingesetzt.

### Schritt 2.2

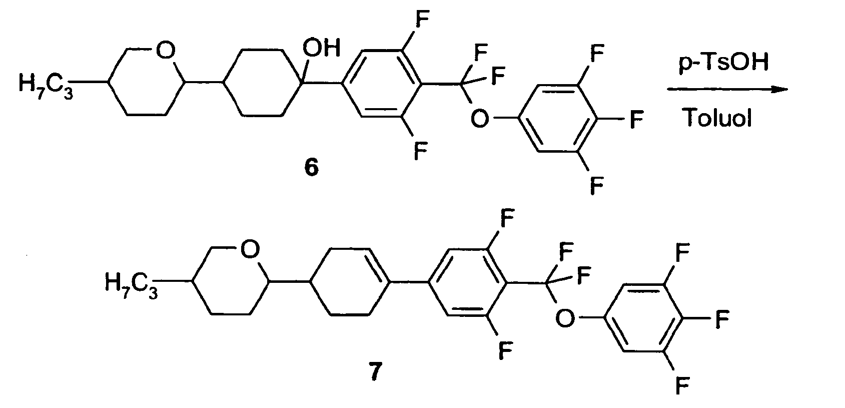

Analog Beispiel 1, Schritt 1.2 wird die Zwischenstufe 6 aus Schritt 2.1 mit p-TsOH zum Cyclohexen 7 eliminiert. Anschließend wird das Lösungsmittel entfernt und der erhaltene Rückstand mit Toluol über Kieselgel gegeben. Kristallisation aus Ethanol und n-Heptan.
K 56 N 121 I
Δε 27
Δn 0,123

Die Verbindungen fallen als Diastereomerengemische der beiden trans-Isomere am Tetrahydropyran an (ca. 1:1, HPLC). Die Werte gelten, soweit nicht anders angegeben, für die entstandenen Diastereomerengemische.

Analog werden die folgenden Verbindungen der Formel 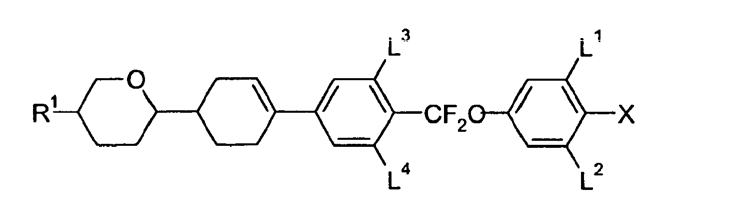 hergestellt:

| R¹ | X | L¹ | L² | L³ | L⁴ | Werte |
|---|---|---|---|---|---|---|
| H | F | H | H | F | F | |
| CH₃ | F | H | H | F | F | |
| C₂H₅ | F | H | H | F | F | |
| n-C₄H₉ | F | H | H | F | F | |
| n-C₅H₁₁ | F | H | H | F | F | |
| n-C₆H₁₃ | F | H | H | F | F | |
| H | F | F | H | F | F | |
| CH₃ | F | F | H | F | F | |
| C₂H₅ | F | F | H | F | F | |
| n-C₃H₇ | F | F | H | F | F | |
| n-C₄H₉ | F | F | H | F | F | |
| n-C₅H₁₁ | F | F | H | F | F | |
| n-C₆H₁₃ | F | F | H | F | F | |
| H | F | F | F | F | F | |
| CH₃ | F | F | F | F | F | |
| C₂H₅ | F | F | F | F | F | K 66 N 96 I; Δε 27; Δn 0,120 |
| n-C₃H₇ | F | F | F | F | F | vgl. Beispiel 2 |
| n-C₄H₉ | F | F | F | F | F | K 32 N 116 I; Δε 26; Δn 0,118 |
| -C₅H₁₁ | F | F | F | F | F | K 62 N 119 I; Δε 25; Δn 0,123 |
| n-C₆H₁₃ | F | F | F | F | F | |
| n-C₇H₁₅ | F | F | F | F | F | |
| H | OCF₃ | H | H | F | F | |
| CH₃ | OCF₃ | H | H | F | F | |
| C₂H₅ | OCF₃ | H | H | F | F | |
| n-C₄H₉ | OCF₃ | H | H | F | F | |
| n-C₅H₁₁ | OCF₃ | H | H | F | F | |
| n-C₆H₁₃ | OCF₃ | H | H | F | F | |
| H | OCF₃ | F | H | F | F | |
| CH₃ | OCF₃ | F | H | F | F | |
| C₂H₅ | OCF₃ | F | H | F | F | |
| n-C₃H₇ | OCF₃ | F | H | F | F | K 5 SmA 110 N 143 I; Δε 22; Δn 0,125 |
| n-C₄H₉ | OCF₃ | F | H | F | F | |
| n-C₅H₁₁ | OCF₃ | F | H | F | F | |
| n-C₆H₁₃ | OCF₃ | F | H | F | F | |
| H | OCF₃ | F | F | F | F | |
| CH₃ | OCF₃ | F | F | F | F | |
| C₂H₅ | OCF₃ | F | F | F | F | |
| n-C₃H₇ | OCF₃ | F | F | F | F | K 43 SmA 105 N 144 I; Δε 23; Δn 0,129 |
| n-C₄H₉ | OCF₃ | F | F | F | F | |
| n-C₅H₁₁ | OCF₃ | F | F | F | F | |
| n-C₆H₁₃ | OCF₃ | F | F | F | F | |
| n-C₇H₁₅ | OCF₃ | F | F | F | F | |
| H | F | H | H | H | H | |
| CH₃ | F | H | H | H | H | |
| C₂H₅ | F | H | H | H | H | |
| n-C₄H₉ | F | H | H | H | H | |
| n-C₅H₁₁ | F | H | H | H | H | |
| n-C₆H₁₃ | F | H | H | H | H | |
| H | F | F | H | H | H | |
| CH₃ | F | F | H | H | H | |
| C₂H₅ | F | F | H | H | H | |
| n-C₃H₇ | F | F | H | H | H | K 44 SmB 142 SmA 179 N 187 I; Δε 18; Δn 0,142 |
| n-C₄H₉ | F | F | H | H | H | |
| n-C₅H₁₁ | F | F | H | H | H | |
| n-C₆H₁₃ | F | F | H | H | H | |
| H | F | F | F | H | H | |
| CH₃ | F | F | F | H | H | |
| C₂H₅ | F | F | F | H | H | |
| n-C₃H₇ | F | F | F | H | H | K68SmB108SmA114N 161 1; Δε 20; Δn 0,140 |
| n-C₄H₉ | F | F | F | H | H | |
| n-C₅H₁₁ | F | F | F | H | H | |
| n-C₆H₁₃ | F | F | F | H | H | |
| n-C₇H₁₅ | F | F | F | H | H | |
| H | OCF₃ | H | H | H | H | |
| CH₃ | OCF₃ | H | H | H | H | |
| C₂H₅ | OCF₃ | H | H | H | H | |
| n-C₄H₉ | OCF₃ | H | H | H | H | |
| n-C₅H₁₁ | OCF₃ | H | H | H | H | |
| n-C₆H₁₃ | OCF₃ | H | H | H | H | |
| H | OCF₃ | F | H | H | H | |
| CH₃ | OCF₃ | F | H | H | H | |
| C₂H₅ | OCF₃ | F | H | H | H | |
| n-C₃H₇ | OCF₃ | F | H | H | H | |
| n-C₄H₉ | OCF₃ | F | H | H | H | |
| n-C₅H₁₁ | OCF₃ | F | H | H | H | |
| n-C₆H₁₃ | OCF₃ | F | H | H | H | |
| H | OCF₃ | F | F | H | H | |
| CH₃ | OCF₃ | F | F | H | H | |
| C₂H₅ | OCF₃ | F | F | H | H | |
| n-C₃H₇ | OCF₃ | F | F | H | H | K 57 SmB 95 SmA 134 N 169 I; Δε 22; An 0,138 |
| n-C₄H₉ | OCF₃ | F | F | H | H | |
| n-C₅H₁₁ | OCF₃ | F | F | H | H | |
| n-C₆H₁₃ | OCF₃ | F | F | H | H | |
| n-C₇H₁₅ | OCF₃ | F | F | H | H | |

### Beispiel 3

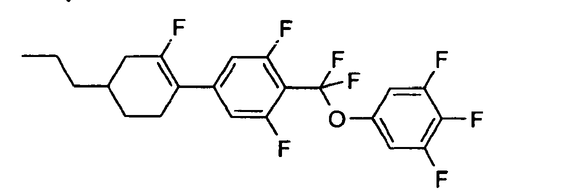

### Schritt 3.1

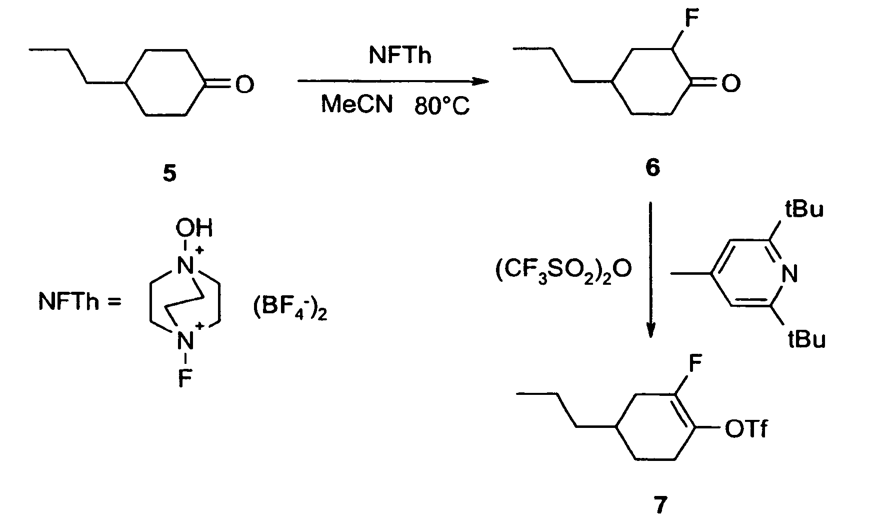

Nach S. Stavber, M. Zupan, Tetrahedron Lett. 1996, 37, 3591-3594 wird das α-fluorierte Keton **6** durch Reaktion mit 1-Fluoro-4-hydroxy-1,4-diazoniabicyclo[2.2.2]-octanbis(tetrafluoroborat) (Accufluor™ NFTh) in Acetonitril hergestellt. Das Fluorketon **6** wird, analog M.A. Hoosain, Tetrahedron Lett. 1997, 38, 49-52, mit Trifluorsulfonsäure-Anhydrid in das Enoltriflat **7** überführt.

Aus dem Baustein **2** (vgl. Beispiel 1), Isopropylmagnesiumchlorid und Trimethylborat mit anschließender saurer Hydrolyse kann die Boronsäure **9** hergestellt werden.

### Schritt 3.2

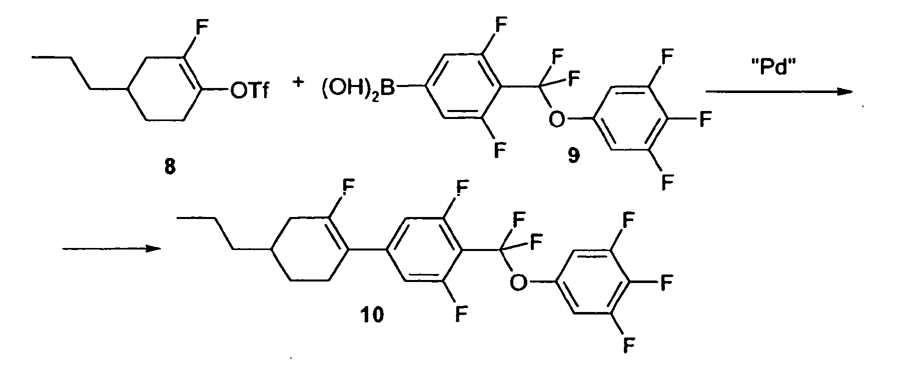

Unter Stickstoff werden 24,5 g (74 mmol) Natriummetaborat-Oktahydrat in 30 ml Wasser vorgelegt und mit 30 ml THF, 700 ml Bis(triphenylphosphin)-palladium(11)chlorid und 0,1 ml 80%igem Hydraziniumhydroxid versetzt. Nach 5 Minuten werden 14,5 g (50 mmol) des Triflats **8** und 17,7 g (50 mmol) der Boronsäure **9** sowie weitere 30 ml THF hinzugefügt. Der Ansatz wird 6 h unter Rückfluss erhitzt. Das abgekühlte Gemisch wird mit 100 ml MTB-Ether verdünnt. Die organische Phase wird eingeengt und der Rückstand an Kieselgel (Toluol) gereinigt. Kristallisation aus Ethanol und n-Heptan.

Analog werden die folgenden Verbindungen der Formel 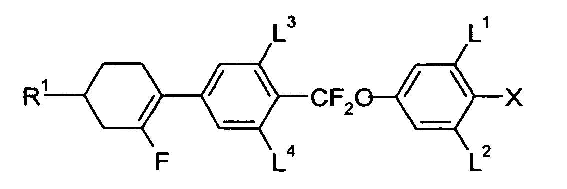 hergestellt.

| R¹ | X | L¹ | L² | L³ | L⁴ |
|---|---|---|---|---|---|
| H | F | H | H | H | H |
| CH₃ | F | H | H | H | H |
| C₂H₅ | F | H | H | H | H |
| C₃H₇ | F | H | H | H | H |
| n-C₄H₉ | F | H | H | H | H |
| n-C₅H₁₁ | F | H | H | H | H |
| n-C₆H₁₃ | F | H | H | H | H |
| H | F | F | H | H | H |
| CHF | F | F | H | H | H |
| C₂H₅ | F | F | H | H | H |
| n-C₃H₇ | F | F | H | H | H |
| n-C₄H₉ | F | F | H | H | H |
| n-C₅H₁₁ | F | F | H | H | H |
| n-C₆H₁₃ | F | F | H | H | H |
| H | F | F | F | H | H |
| CH₃ | F | F | F | H | H |
| n-C₄H₉ | F | F | F | H | H |
| n-C₅H₁₁ | F | F | F | H | H |
| n-C₆H₁₃ | F | F | F | H | H |
| H | OCF₃ | H | H | H | H |
| CH₃ | OCF₃ | H | H | H | H |
| C₂H₅ | OCF₃ | H | H | H | H |
| n-C₃H₇ | OCF₃ | H | H | H | H |
| n-C₄H₉ | OCF₃ | H | H | H | H |
| n-C₅H₁₁ | OCF₃ | H | H | H | H |
| n-C₆H₁₃ | OCF₃ | H | H | H | H |
| H | OCF₃ | F | H | H | H |
| CH₃ | OCF₃ | F | H | H | H |
| C₂H₅ | OCF₃ | F | H | H | H |
| n-C₃H₇ | OCF₃ | F | H | H | H |
| n-C₄H₉ | OCF₃ | F | H | H | H |
| n-C₅H₁₁ | OCF₃ | F | H | H | H |
| n-C₆H₁₃ | OCF₃ | F | H | H | H |
| H | OCF₃ | F | F | H | H |
| CH₃ | OCF₃ | F | F | H | H |
| C₂H₅ | OCF₃ | F | F | H | H |
| n-C₃H₇ | OCF₃ | F | F | H | H |
| n-C₄H₉ | OCF₃ | F | F | H | H |
| n-C₅H₁₁ | OCF₃ | F | F | H | H |
| n-C₆H₁₃ | OCF₃ | F | F | H | H |
| H | CN | H | H | H | H |
| CH₃ | CN | H | H | H | H |
| C₂H₅ | CN | H | H | H | H |
| n-C₃H₇ | CN | H | H | H | H |
| n-C₄H₉ | CN | H | H | H | H |
| n-C₅H₁₁ | CN | H | H | H | H |
| n-C₆H₁₃ | CN | H | H | H | H |
| H | CN | F | H | H | H |
| CH₃ | CN | F | H | H | H |
| C₂H₅ | CN | F | H | H | H |
| n-C₃H₇ | CN | F | H | H | H |
| n-C₄H₉ | CN | F | H | H | H |
| n-C₅H₁₁ | CN | F | H | H | H |
| n-C₆H₁₃ | CN | F | H | H | H |
| H | CN | F | F | H | H |
| CH₃ | CN | F | F | H | H |
| C₂H₅ | CN | F | F | H | H |
| n-C₃H₇ | CN | F | F | H | H |
| n-C₄H₉ | CN | F | F | H | H |
| n-C₅H₁₁ | CN | F | F | H | H |
| n-C₆H₁₃ | CN | F | F | H | H |
| H | F | H | H | F | H |
| CH₃ | F | H | H | F | H |
| C₂H₅ | F | H | H | F | H |
| C₃H₇ | F | H | H | F | H |
| n-C₄H₉ | F | H | H | F | H |
| n-C₅H₁₁ | F | H | H | F | H |
| n-C₆H₁₃ | F | H | H | F | H |
| H | F | F | H | F | H |
| CH₃ | F | F | H | F | H |
| C₂H₅ | F | F | H | F | H |
| n-C₃H₇ | F | F | H | F | H |
| n-C₄H₉ | F | F | H | F | H |
| n-C₅H₁₁ | F | F | H | F | H |
| n-C₆H₁₃ | F | F | H | F | H |
| H | F | F | F | F | H |
| CH₃ | F | F | F | F | H |
| C₂H₅ | F | F | F | F | H |
| n-C₃H₇ | F | F | F | F | H |
| n-C₄H₉ | F | F | F | F | H |
| n-C₅H₁₁ | F | F | F | F | H |
| n-C₆H₁₃ | F | F | F | F | H |
| H | OCF₃ | H | H | F | H |
| CH₃ | OCF₃ | H | H | F | H |
| C₂H₅ | OCF₃ | H | H | F | H |
| n-C₃H₇ | OCF₃ | H | H | F | H |
| n-C₄H₉ | OCF₃ | H | H | F | H |
| n-C₅H₁₁ | OCF₃ | H | H | F | H |
| n-C₆H₁₁ | OCF₃ | H | H | F | H |
| H | OCF₃ | F | H | F | H |
| CH₃ | OCF₃ | F | H | F | H |
| C₂H₅ | OCF₃ | F | H | F | H |
| n-C₃H₇ | OCF₃ | F | H | F | H |
| n-C₄H₉ | OCF₃ | F | H | F | H |
| n-C₅H₁₁ | OCF₃ | F | H | F | H |
| n-C₆H₁₃ | OCF₃ | F | H | F | H |
| H | OCF₃ | F | F | F | H |
| CH₃ | OCF₃ | F | F | F | H |
| C₂H₅ | OCF₃ | F | F | F | H |
| n-C₃H₇ | OCF₃ | F | F | F | H |
| n-C₄H₉ | OCF₃ | F | F | F | H |
| n-C₅H₁₁ | OCF₃ | F | F | F | H |
| n-C₆H₁₃ | OCF₃ | F | F | F | H |
| H | CN | H | H | F | H |
| CH₃ | CN | H | H | F | H |
| C₂H₅ | CN | H | H | F | H |
| n-C₃H₇ | CN | H | H | F | H |
| n-C₄H₉ | CN | H | H | F | H |
| n-C₅H₁₁ | CN | H | H | F | H |
| n-C₆H₁₃ | CN | H | H | F | H |
| H | CN | F | H | F | H |
| CH₃ | CN | F | H | F | H |
| C₂H₅ | CN | F | H | F | H |
| n-C₃H₇ | CN | F | H | F | H |
| n-C₄H₉ | CN | F | H | F | H |
| n-C₅H₁₁ | CN | F | H | F | H |
| n-C₆H₁₃ | CN | F | H | F | H |
| H | CN | F | F | F | H |
| CH₃ | CN | F | F | F | H |
| C₂H₅ | CN | F | F | F | H |
| n-C₃H₇ | CN | F | F | F | H |
| n-C₄H₉ | CN | F | F | F | H |
| n-C₅H₁₁ | CN | F | F | F | H |
| n-C₆H₁₃ | CN | F | F | F | H |
| H | F | H | H | F | F |
| CH₃ | F | H | H | F | F |
| C₂H₅ | F | H | H | F | F |
| n-C₄H₉ | F | H | H | F | F |
| n-C₅H₁₁ | F | H | H | F | F |
| n-C₆H₁₃ | F | H | H | F | F |
| H | F | F | H | F | F |
| CH₃ | F | F | H | F | F |
| C₂H₅ | F | F | H | F | F |
| n-C₃H₇ | F | F | H | F | F |
| n-C₄H₉ | F | F | H | F | F |
| n-C₅H₁₁ | F | F | H | F | F |
| n-C₆H₁₃ | F | F | H | F | F |
| H | F | F | F | F | F |
| CH₃ | F | F | F | F | F |
| C₂H₅ | F | F | F | F | F |
| C₂H₅ | F | F | F | F | F |
| n-C₃H₇ | F | F | F | F | F |
| n-C₄H₉ | F | F | F | F | F |
| n-C₅H₁₁ | F | F | F | F | F |
| n-C₆H₁₃ | F | F | F | F | F |
| H | OCF₃ | H | H | F | F |
| CH₃ | OCF₃ | H | H | F | F |
| C₂H₅ | OCF₃ | H | H | F | F |
| n-C₃H₇ | OCF₃ | H | H | F | F |
| n-C₄H₉ | OCF₃ | H | H | F | F |
| n-C₅H₁₁ | OCF₃ | H | H | F | F |
| n-C₆H₁₃ | OCF₃ | H | H | F | F |
| H | OCF₃ | F | H | F | F |
| CH₃ | OCF₃ | F | H | F | F |
| C₂H₅ | OCF₃ | F | H | F | F |
| n-C₃H₇ | OCF₃ | F | H | F | F |
| n-C₄H₉ | OCF₃ | F | H | F | F |
| n-C₅H₁₁ | OCF₃ | F | H | F | F |
| n-C₆H₁₃ | OCF₃ | F | H | F | F |
| H | OCF₃ | F | F | F | F |
| CH₃ | OCF₃ | F | F | F | F |
| C₂H₅ | OCF₃ | F | F | F | F |
| n-C₃H₇ | OCF₃ | F | F | F | F |
| n-C₄H₉ | OCF₃ | F | F | F | F |
| n-C₅H₁₁ | OCF₃ | F | F | F | F |
| n-C₆H₁₃ | OCF₃ | F | F | F | F |
| H | CN | H | H | F | F |
| CH₃ | CN | H | H | F | F |
| C₂H₅ | CN | H | H | F | F |
| n-C₃H₇ | CN | H | H | F | F |
| n-C₄H₉ | CN | H | H | F | F |
| n-C₅H₁₁ | CN | H | H | F | F |
| n-C₆H₁₃ | CN | H | H | F | F |
| H | CN | F | H | F | F |
| CH₃ | CN | F | H | F | F |
| C₂H₅ | CN | F | H | F | F |
| n-C₃H₇ | CN | F | H | F | F |
| n-C₄H₉ | CN | F | H | F | F |
| n-C₅H₁₁ | CN | F | H | F | F |
| n-C₆H₁₃ | CN | F | H | F | F |
| H | CN | F | F | F | F |
| CH₃ | CN | F | F | F | F |
| C₂H₅ | CN | F | F | F | F |
| n-C₃H₇ | CN | F | F | F | F |
| n-C₄H₉ | CN | F | F | F | F |
| n-C₅H₁₁ | CN | F | F | F | F |
| n-C₆H₁₃ | CN | F | F | F | F |

### Beispiel 4

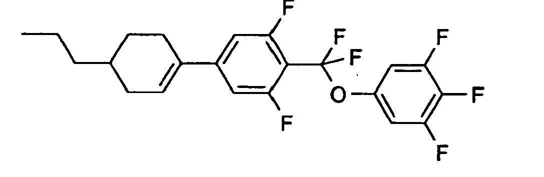

### Schritt 4.1

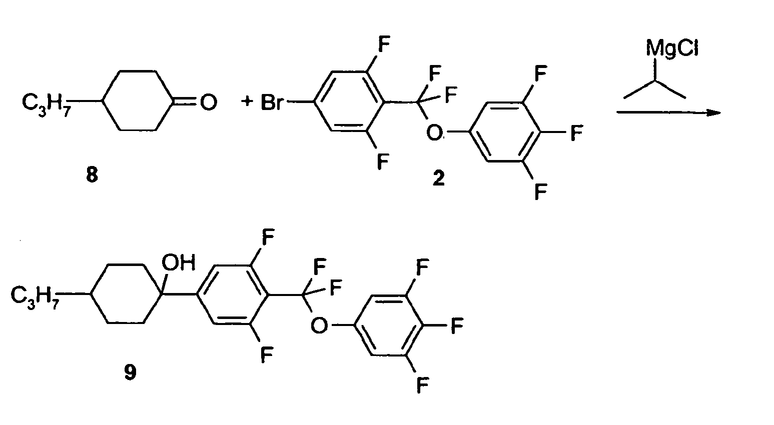

96 ml (190 mmol) einer 2 M Lösung von Isopropylmagnesiumchlorid in THF werden unter Stickstoff bei 20°C mit einer Lösung von 62.1 g (160 mmol) des Bromids **2** in 300 ml THF versetzt. Nach 1 h werden 190 mmol des Ketons **11** gelöst in 200 ml THF bei ebenfalls 20°C zugegeben. Die Aufarbeitung erfolgt analog Schritt 1.1. Die Substanz **12** wird ohne weitere Reinigung in der Folgestufe eingesetzt.

### Schritt 4.2

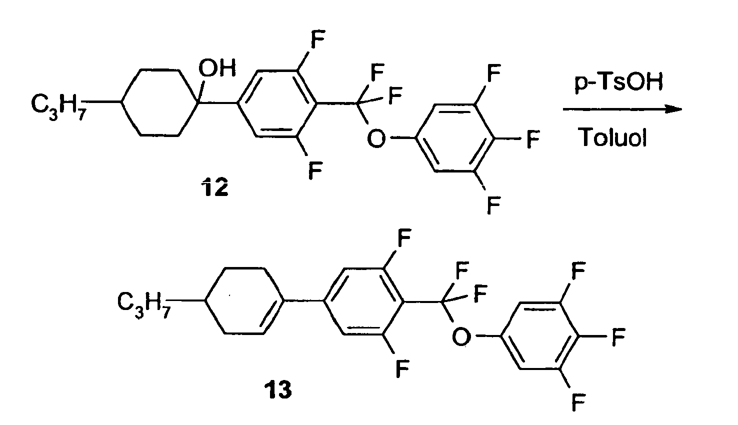

Die Umsetzung und Reinigung erfolgt analog Schritt 1.2.
K22 I
Δε 21
Δn 0,095
γ1 68 mPa·s

Analog zu Beispiel 4 werden die folgenden Verbindungen der Formel 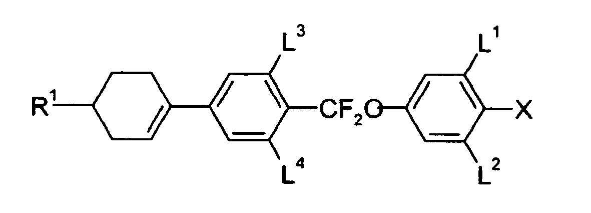 mit L³ = F hergestellt:

| R¹ | X | L¹ | L² | L³ | L⁴ | Werte |
|---|---|---|---|---|---|---|
| H | F | H | H | F | H | |
| CH₃ | F | H | H | F | H | |
| C₂H₅ | F | H | H | F | H | |
| C₃H₇ | F | H | H | F | H | |
| n-C₄H₉ | F | H | H | F | H | |
| n-C₅H₁₁ | F | H | H | F | H | |
| n-C₆H₁₃ | F | H | H | F | H | |
| H | F | F | H | F | H | |
| CH₃ | F | F | H | F | H | |
| C₂H₅ | F | F | H | F | H | |
| n-C₃H₇ | F | F | H | F | H | K 28 N (-7) I; Δε 17; Δn 0,106; γ₁ 112 mPa·s |
| n-C₄H₉ | F | F | H | F | H | |
| n-C₅H₁₁ | F | F | H | F | H | |
| n-C₆H₁₃ | F | F | H | F | H | |
| H | F | F | F | F | H | |
| CH₃ | F | F | F | F | H | |
| C₂H₅ | F | F | F | F | H | |
| n-C₃H₇ | F | F | F | F | H | K 28 N (-7) I; Δε 17; Δn 0,106; γ1 112 mPa·s |
| n-C₄H₉ | F | F | F | F | H | |
| n-C₅H₁₁ | F | F | F | F | H | |
| n-C₆H₁₃ | F | F | F | F | H | |
| H | OCF₃ | H | H | F | H | |
| CH₃ | OCF₃ | H | H | F | H | |
| C₂H₅ | OCF₃ | H | H | F | H | |
| C₃H₇ | OCF₃ | H | H | F | H | |
| n-C₄H₉ | OCF₃ | H | H | F | H | |
| n-C₅H₁₁ | OCF₃ | H | H | F | H | |
| n-C₆H₁₃ | OCF₃ | H | H | F | H | |
| H | OCF₃ | F | H | F | H | |
| CH₃ | OCF₃ | F | H | F | H | |
| C₂H₅ | OCF₃ | F | H | F | H | |
| n-C₃H₇ | OCF₃ | F | H | F | H | |
| n-C₄H₉ | OCF₃ | F | H | F | H | |
| n-C₅H₁₁ | OCF₃ | F | H | F | H | |
| n-C₆H₁₃ | OCF₃ | F | H | F | H | |
| H | OCF₃ | F | F | F | H | |
| CH₃ | OCF₃ | F | F | F | H | |
| C₂H₅ | OCF₃ | F | F | F | H | |
| n-C₃H₇ | OCF₃ | F | F | F | H | |
| n-C₄H₉ | OCF₃ | F | F | F | H | |
| n-C₅H₁₁ | OCF₃ | F | F | F | H | |
| n-C₆H₁₃ | OCF₃ | F | F | F | H | |
| H | Cl | H | H | F | H | |
| CH₃ | Cl | H | H | F | H | |
| C₂H₅ | Cl | H | H | F | H | |
| C₃H₇ | Cl | H | H | F | H | |
| n-C₄H₉ | Cl | H | H | F | H | |
| n-C₅H₁₁ | Cl | H | H | F | H | |
| n-C₆H₁₃ | Cl | H | H | F | H | |
| H | Cl | F | H | F | H | |
| CH₃ | Cl | F | H | F | H | |
| C₂H₅ | Cl | F | H | F | H | |
| n-C₃H₇ | Cl | F | H | F | H | |
| n-C₄H₉ | Cl | F | H | F | H | |
| n-C₅H₁₁ | Cl | F | H | F | H | |
| n-C₆H₁₃ | Cl | F | H | F | H | |
| H | Cl | F | F | F | H | |
| CH₃ | Cl | F | F | F | H | |
| C₂H₅ | Cl | F | F | F | H | |
| n-C₃H₇ | Cl | F | F | F | H | |
| n-C₄H₉ | Cl | F | F | F | H | |
| n-C₅H₁₁ | Cl | F | F | F | H | |
| n-C₆H₁₃ | Cl | F | F | F | H | |
| H | OCHF₂ | H | H | F | H | |
| CH₃ | OCHF₂ | H | H | F | H | |
| C₂H₅ | OCHF₂ | H | H | F | H | |
| n-C₃H₇ | OCHF₂ | H | H | F | H | |
| n-C₄H₉ | OCHF₂ | H | H | F | H | |
| n-C₅H₁₁ | OCHF₂ | H | H | F | H | |
| n-C₆H₁₃ | OCHF₂ | H | H | F | H | |
| H | OCHF₂ | F | H | F | H | |
| CH₃ | OCHF₂ | F | H | F | H | |
| C₂H₅ | OCHF₂ | F | H | F | H | |
| n-C₃H₇ | OCHF₂ | F | H | | H | |
| -C₄H₉ | OCHF₂ | F | H | F | H | |
| n-C₅H₁₁ | OCHF₂ | F | H | F | H | |
| n-C₆H₁₃ | OCHF₂ | F | H | F | H | |
| H | OCHF₂ | F | F | F | H | |
| CH₃ | OCHF₂ | F | F | F | H | |
| C₂H₅ | OCHF₂ | F | F | F | H | |
| n-C₃H₇ | OCHF₂ | F | F | F | H | |
| n-C₄H₉ | OCHF₂ | F | F | F | H | |
| n-C₅H₁₁ | OCHF₂ | F | F | F | H | |
| n-C₆H₁₃ | OCHF₂ | F | F | F | H | |
| H | OCHFCF₃ | H | H | F | H | |
| CH₃ | OCHFCF₃ | H | H | F | H | |
| C₂H₅ | OCHFCF₃ | H | H | F | H | |
| n-C₃H₇ | OCHFCF₃ | H | H | F | H | |
| n-C₄H₉ | OCHFCF₃ | H | H | F | H | |
| n-C₅H₁₁ | OCHFCF₃ | H | H | F | H | |
| n-C₆H₁₃ | OCHFCF₃ | H | H | F | H | |
| H | OCHFCF₃ | F | H | F | H | |
| CH₃ | OCHFCF₃ | F | H | F | H | |
| C₂H₅ | OCHFCF₃ | F | H | F | H | |
| n-C₃H₇ | OCHFCF₃ | F | H | F | H | |
| n-C₄H₉ | OCHFCF₃ | F | H | F | H | |
| n-C₅H₁₁ | OCHFCF₃ | F | H | F | H | |
| n-C₆H₁₃ | OCHFCF₃ | F | H | F | H | |
| H | OCHFCF₃ | F | F | F | H | |
| CH₃ | OCHFCF₃ | F | F | F | H | |
| C₂H₅ | OCHFCF₃ | F | F | F | H | |
| n-C₃H₇ | OCHFCF₃ | F | F | F | H | |
| n-C₄H₉ | OCHFCF₃ | F | F | F | H | |
| n-C₅H₁₁ | OCHFCF₃ | F | F | F | H | |
| n-C₆H₁₃ | OCHFCF₃ | F | F | F | H | |
| H | OCHFCF₃ | H | H | F | H | |
| CH₃ | OCHFCF₃ | H | H | F | H | |
| C₂H₅ | OCHFCF₃ | H | H | F | H | |
| n-C₃H₇ | OCHFCF₃ | H | H | F | H | |
| n-C₄H₉ | OCHFCF₃ | H | H | F | H | |
| n-C₅H₁₁ | OCHFCF₃ | H | H | F | H | |
| n-C₆H₁₃ | OCHFCF₃ | H | H | F | H | |
| H | OCHFCF₃ | F | H | F | H | |
| CH₃ | OCHFCF₃ | F | H | F | H | |
| C₂H₅ | OCHFCF₃ | F | H | F | H | |
| n-C₃H₇ | OCHFCF₃ | F | H | F | H | |
| n-C₄H₉ | OCHFCF₃ | F | H | F | H | |
| n-C₅H₁₁ | OCHFCF₃ | F | H | F | H | |
| n-C₆H₁₃ | OCHFCF₃ | F | H | F | H | |
| H | OCHFCF₃ | F | F | F | H | |
| CH₃ | OCHFCF₃ | F | F | F | H | |
| C₂H₅ | OCHFCF₃ | F | F | F | H | |
| n-C₃H₇ | OCHFCF₃ | F | F | F | H | |
| n-C₄H₉ | OCHFCF₃ | F | F | F | H | |
| n-C₅H₁₁ | OCHFCF₃ | F | F | F | H | |
| n-C₆H₁₃ | OCHFCF₃ | F | F | F | H | |
| H | NCS | H | H | F | H | |
| CH₃ | NCS | H | H | F | H | |
| C₂H₅ | NCS | H | H | F | H | |
| n-C₃H₇ | NCS | H | H | F | H | |
| n-C₄H₉ | NCS | H | H | F | H | |
| n-C₅H₁₁ | NCS | H | H | F | H | |
| n-C₆H₁₃ | NCS | H | H | F | H | |
| H | NCS | F | H | F | H | |
| CH₃ | NCS | F | H | F | H | |
| C₂H₅ | NCS | F | H | F | H | |
| n-C₃H₇ | NCS | F | H | F | H | |
| n-C₄H₉ | NCS | F | H | F | H | |
| n-C₅H₁₁ | NCS | F | H | F | H | |
| n-C₆H₁₃ | NCS | F | H | F | H | |
| H | NCS | F | F | F | H | |
| CH₃ | NCS | F | F | F | H | |
| C₂H₅ | NCS | F | F | F | H | |
| n-C₃H₇ | NCS | F | F | F | H | |
| n-C₄H₉ | NCS | F | F | F | H | |
| n-C₅H₁₁ | NCS | F | F | F | H | |
| n-C₆H₁₃ | NCS | F | F | F | H | |
| n-C₆H₁₃ | C₃H₇ | F | F | F | H | |
| H | SF₅ | H | H | F | H | |
| CH₃ | SF₅ | H | H | F | H | |
| C₂H₅ | SF₅ | H | H | F | H | |
| n-C₃H₇ | SF₅ | H | H | F | H | |
| n-C₄H₉ | SF₅ | H | H | F | H | |
| n-C₅H₁₁ | SF₅ | H | H | F | H | |
| n-C₆H₁₃ | SF₅ | H | H | F | H | |
| H | SF₅ | F | H | F | H | |
| CH₃ | SF₅ | F | H | F | H | |
| C₂H₅ | SF₅ | F | H | F | H | |
| n-C₃H₇ | SF₅ | F | H | F | H | |
| n-C₄H₉ | SF₅ | F | H | F | H | |
| n-C₅H₁₁ | SF₅ | F | H | F | H | |
| n-C₆H₁₃ | SF₅ | F | H | F | H | |
| H | SF₅ | F | F | F | H | |
| CH₃ | SF₅ | F | F | F | H | |
| C₂H₅ | SF₅ | F | F | F | H | |
| n-C₃H₇ | SF₅ | F | F | F | H | |
| n-C₄H₉ | SF₅ | F | F | F | H | |
| n-C₅H₁₁ | SF₅ | F | F | F | H | |
| n-C₆H₁₃ | SF₅ | F | F | F | H | |
| H | CN | H | H | F | H | |
| CH₃ | CN | H | H | F | H | |
| C₂H₅ | CN | H | H | F | H | |
| n-C₃H₇ | CN | H | H | F | H | |
| n-C₄H₉ | CN | H | H | F | H | |
| n-C₅H₁₁ | CN | H | H | F | H | |
| n-C₆H₁₃ | CN | H | H | F | H | |
| H | CN | F | H | F | H | |
| CH₃ | CN | F | H | F | H | |
| C₂H₅ | CN | F | H | F | H | |
| n-C₃H₇ | CN | F | H | F | H | |
| n-C₄H₉ | CN | F | H | F | H | |
| n-C₅H₁₁ | CN | F | H | F | H | |
| n-C₆H₁₃ | CN | F | H | F | H | |
| H | CN | F | F | F | H | |
| CH₃ | CN | F | F | F | H | |
| C₂H₅ | CN | F | F | F | H | |
| n-C₃H₇ | CN | F | F | F | H | |
| n-C₄H₉ | CN | F | F | F | H | |
| n-C₅H₁₁ | CN | F | F | F | H | |
| n-C₆H₁₃ | CN | F | F | F | H | |
| H | F | H | H | F | F | |
| CH₃ | F | H | H | F | F | |
| C₂H₅ | F | H | H | F | F | |
| n-C₄H₉ | F | H | H | F | F | |
| n-C₅H₁₁ | F | H | H | F | F | |
| n-C₆H₁₃ | F | H | H | F | F | |
| H | F | F | H | F | F | |
| CH₃ | F | F | H | F | F | |
| C₂H₅ | F | F | H | F | F | |
| n-C₃H₇ | F | F | H | F | F | |
| n-C₄H₉ | F | F | H | F | F | |
| n-C₅H₁₁ | F | F | H | F | F | |
| n-C₆H₁₃ | F | F | H | F | F | |
| H | F | F | F | F | F | |
| CH₃ | F | F | F | F | F | |
| -CH=CH₂ | F | F | F | F | F | |
| C₂H₅ | F | F | F | F | F | Δε 19; Δn 0,080; γ₁ 58 mPa·s |
| n-C₃H₇ | F | F | F | F | F | vgl. Beispiel 4 |
| n-C₄H₉ | F | F | F | F | F | Δε 20; Δn 0,082; γ₁ 76 mPa·s |
| n-C₆H₁₁ | F | F | F | F | F | Δε 19; Δn 0,085; γ₁ 115 mPa·s |
| n-C₆H₁₃ | F | F | F | F | F | |
| n-C₇H₁₅ | F | F | F | F | F | |
| H | OCF₃ | H | H | F | F | |
| CH₃ | OCF₃ | H | H | F | F | |
| C₂H₅ | OCF₃ | H | H | F | F | |
| n-C₄H₉ | OCF₃ | H | H | F | F | |
| n-C₅H₁₁ | OCF₃ | H | H | F | F | |
| n-C₆H₁₃ | OCF₃ | H | H | F | F | |
| H | OCF₃ | F | H | F | F | |
| CH₃ | OCF₃ | F | H | F | F | |
| C₂H₅ | OCF₃ | F | H | F | F | |
| n-C₃H₇ | OCF₃ | F | H | F | F | |
| n-C₄H₉ | OCF₃ | F | H | F | F | |
| n-C₅H₁₁ | OCF₃ | F | H | F | F | |
| n-C₆H₁₃ | OCF₃ | F | H | F | F | |
| H | OCF₃ | F | F | F | F | |
| CH₃ | OCF₃ | F | F | F | F | |
| -CH=CH₂ | OCF₃ | F | F | F | F | |
| C₂H₅ | OCF₃ | F | F | F | F | |
| n-C₃H₇ | OCF₃ | F | F | F | F | |
| n-C₄H₉ | OCF₃ | F | F | F | F | |
| n-C₅H₁₁ | OCF₃ | F | F | F | F | |
| n-C₆H₁₃ | OCF₃ | F | F | F | F | |
| n-C₇H₁₅ | OCF₃ | F | F | F | F | |
| H | Cl | H | H | F | F | |
| CH₃ | Cl | H | H | F | F | |
| C₂H₅ | Cl | H | H | F | F | |
| C₃H₇ | Cl | H | H | F | F | K 49 N (44) I; Δε 13; Δn 0,140; γ₁ 164 mPa·s |
| n-C₄H₉ | Cl | H | H | F | F | |
| n-C₅H₁₁ | Cl | H | H | F | F | |
| n-C₆H₁₃ | Cl | H | H | F | F | |
| H | Cl | F | H | F | F | |
| CH₃ | Cl | F | H | F | F | |
| C₂H₅ | Cl | F | H | F | F | |
| n-C₃H₇ | Cl | F | H | F | F | K 38 N (23) I; Δε 16; Δn 0,128; γ₁ 141 mPa·s |
| n-C₄H₉ | Cl | F | H | F | F | |
| n-C₅H₁₁ | Cl | F | H | F | F | |
| n-C₆H₁₃ | Cl | F | H | F | F | |
| H | Cl | F | F | F | F | |
| CH₃ | Cl | F | F | F | F | |
| C₂H₅ | Cl | F | F | F | F | |
| n-C₃H₇ | Cl | F | F | F | F | |
| n-C₄H₉ | Cl | F | F | F | F | |
| n-C₅H₁₁ | Cl | F | F | F | F | |
| n-C₆H₁₃ | Cl | F | F | F | F | |
| H | CN | H | H | F | F | |
| CH₃ | CN | H | H | F | F | |
| C₂H₅ | CN | H | H | F | F | |
| n-C₃H₇ | CN | H | H | F | F | |
| n-C₄H₉ | CN | H | H | F | F | |
| n-C₅H₁₁ | CN | H | H | F | F | |
| n-C₆H₁₃ | CN | H | H | F | F | |
| H | CN | F | H | F | F | |
| CH₃ | CN | F | H | F | F | |
| C₂H₅ | CN | F | H | F | F | |
| n-C₃H₇ | CN | F | H | F | F | |
| n-C₄H₉ | CN | F | H | F | F | |
| n-C₅H₁₁ | CN | F | H | F | F | |
| n-C₆H₁₃ | CN | F | H | F | F | |
| H | CN | F | F | F | F | |
| CH₃ | CN | F | F | F | F | |
| C₂H₅ | CN | F | F | F | F | |
| n-C₃H₇ | CN | F | F | F | F | |
| n-C₄H₉ | CN | F | F | F | F | |
| n-C₅H₁₁ | CN | F | F | F | F | |
| n-C₆H₁₃ | CN | F | F | F | F | |
| n-C₇H₁₅ | CN | F | F | F | F | |

Verbindungen mit L³=L⁴=H werden vorzugsweise nach Beispiel 5 hergestellt:

### Beispiel 5

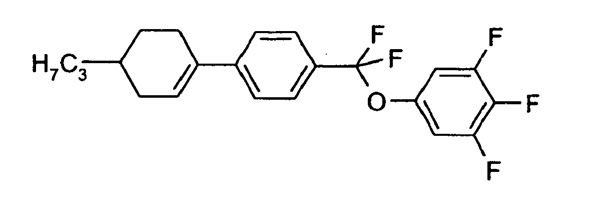

### Schritt 5.1

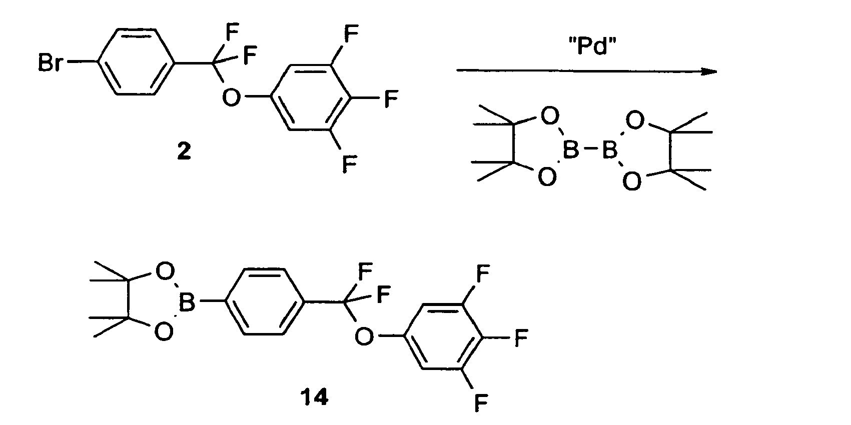

Unter Stickstoff werden 15,0 g (40 mmol) des Bromids 2 in 110 ml Dioxan gelöst und mit 16,2 g (60 mmol) Bis-(pinacolato)-dibor versetzt.

Anschließend werden 12,5 g (130 mmol) Kaliumacetat und 950 mg PdCl₂-dppf (dppf: 1,1'-Bis(diphenylphosphanyl)ferrocen) hinzugefügt. Der Ansatz wird 4 h bei 100°C gerührt. Der abgekühlte Ansatz wird mit 100 ml MTB-Ether verdünnt und mit 70 ml Wasser versetzt. Die wässrige Phase wird mit MTB-Ether extrahiert. Die vereinigten organischen Phasen werden mit Wasser und gesättigter Kochsalzlösung gewaschen, getrocknet und eingeengt. Der Rückstand wird aus Ethanol bei -30°C kristallisiert.

### Schritt 5.2

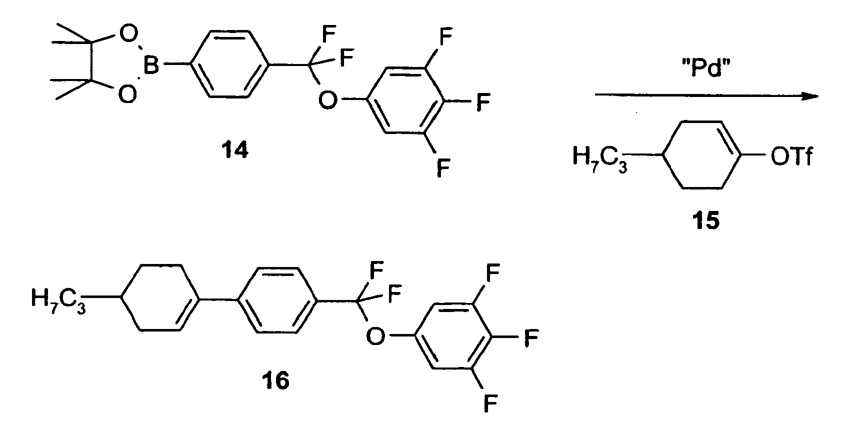

14,5 g Natriummetaborat-Octahydrat in 20 ml Wasser vorgelegt und mit 490 mg Bis(triphenylphosphin)palladium(II)chlorid, 30 ml THF und Hydraziniumhydroxid versetzt. Nach 5 min werden 9,6 g (30 mmol) des Triflats 15 und 14,0 g des Boronesters 14 in den Ansatz gegeben. Nach 15 h bei 70°C wird der Ansatz mit Wasser verdünnt. Die wässrige Phase wird mit MTB-Ether extrahiert. Die vereinigten organischen Phasen werden eingeengt. Der Rückstand wird über Kieselgel filtriert (n-Heptan) und anschließend aus Ethanol und aus n-Heptan kristallisiert.
K 62 N (24) I
Δε 15
An 0,122
γ1 79 mPa·s

Analog zu Beispiel 5 werden die folgenden Verbindungen der Formel 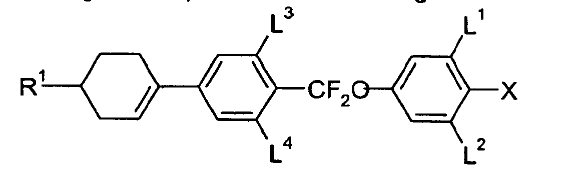 mit L³, L⁴ = H hergestellt:

| R¹ | X | L¹ | L² | L³ | L⁴ | Werte |
|---|---|---|---|---|---|---|
| H | F | H | H | H | H | |
| CH₃ | F | H | H | H | H | |
| C₂H₅ | F | H | H | H | H | |
| C₃H₇ | F | H | H | H | H | |
| n-C₄H₉ | F | H | H | H | H | |
| n-C₅H₁₁ | F | H | H | H | H | |
| n-C₆H₁₃ | F | H | H | H | H | |
| H | F | F | H | H | H | |
| CH₃ | F | F | H | H | H | |
| C₂H₅ | F | F | H | H | H | |
| n-C₃H₇ | F | F | H | H | H | |
| n-C₄H₉ | F | F | H | H | H | |
| n-C₅H₁₁ | F | F | H | H | H | |
| n-C₆H₁₃ | F | F | H | H | H | |
| H | F | F | F | H | H | |
| CH₃ | F | F | F | H | H | |
| C₂H₅ | F | F | F | H | H | |
| n-C₃H₇ | F | F | F | H | H | vgl. Beispiel 5 |
| n-C₄H₉ | F | F | F | H | H | |
| n-C₅H₁₁ | F | F | F | H | H | |
| n-C₆H₁₃ | F | F | F | H | H | |
| H | OCF₃ | H | H | H | H | |
| CH₃ | OCF₃ | H | H | H | H | |
| C₂H₅ | OCF₃ | H | H | H | H | |
| n-C₃H₇ | OCF₃ | H | H | H | H | |
| n-C₄H₉ | OCF₃ | H | H | H | H | |
| n-C₅H₁₁ | OCF₃ | H | H | H | H | |
| n-C₆H₁₃ | OCF₃ | H | H | H | H | |
| H | OCF₃ | F | H | H | H | |
| CH₃ | OCF₃ | F | H | H | H | |
| C₂H₅ | OCF₃ | F | H | H | H | |
| n-C₃H₇ | OCF₃ | F | H | H | H | |
| n-C₄H₉ | OCF₃ | F | H | H | H | |
| n-C₅H₁₁ | OCF₃ | F | H | H | H | |
| n-C₆H₁₃ | OCF₃ | F | H | H | H | |
| H | OCF₃ | F | F | H | H | |
| CH₃ | OCF₃ | F | F | H | H | |
| C₂H₅ | OCF₃ | F | F | H | H | |
| n-C₃H₇ | OCF₃ | F | F | H | H | |
| n-C₄H₉ | OCF₃ | F | F | H | H | |
| n-C₅H₁₁ | OCF₃ | F | F | H | H | |
| n-C₆H₁₃ | OCF₃ | F | F | H | H | |
| H | CN | H | H | H | H | |
| CH₃ | CN | H | H | H | H | |
| C₂H₅ | CN | H | H | H | H | |
| n-C₃H₇ | CN | H | H | H | H | |
| n-C₄H₉ | CN | H | H | H | H | |
| n-C₅H₁₁ | CN | H | H | H | H | |
| n-C₆H₁₃ | CN | H | H | H | H | |
| H | CN | F | H | H | H | |
| CH₃ | CN | F | H | H | H | |
| C₂H₅ | CN | F | H | H | H | |
| n-C₃H₇ | CN | F | H | H | H | |
| n-C₄H9 | CN | F | H | H | H | |
| n-C₅H₁₁ | CN | F | H | H | H | |
| n-C₆H₁₃ | CN | F | H | H | H | |
| H | CN | F | F | H | H | |
| CH₃ | CN | F | F | H | H | |
| C₂H₅ | CN | F | F | H | H | |
| n-C₃H₇ | CN | F | F | H | H | |
| n-C₄H₉ | CN | F | F | H | H | |
| n-C₅H₁₁ | CN | F | F | H | H | |
| n-C₆H₁₃ | CN | F | F | H | H | |

### Beispiel 6

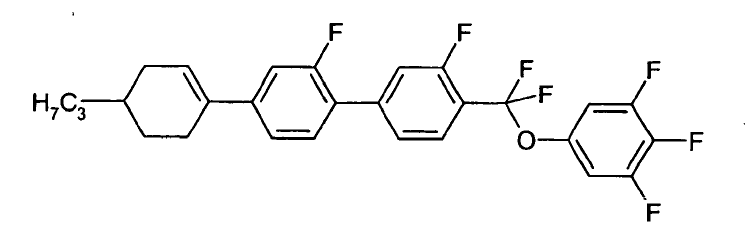

### Schritt 6.1

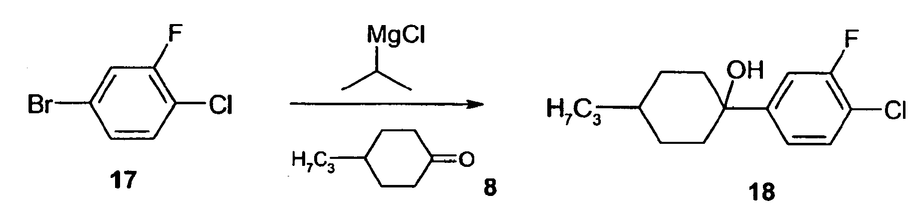

Die Reaktion wird analog Beispiel 4, Schritt 4.1 durchgeführt.

### Schritt 6.2

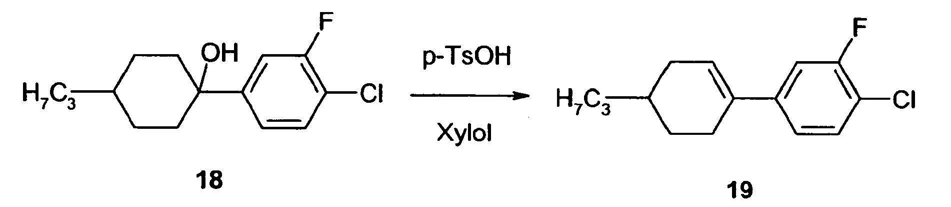

Die Umsetzung und Reinigung erfolgt analog Schritt 1.2.

### Schritt 6.3

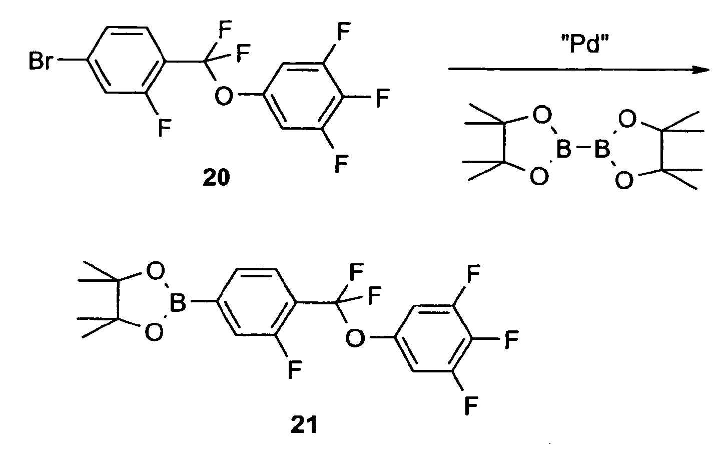

Analog zu Verbindung 14 aus Beispiel 5, Schritt 5.1 wird die Boronsäure als zweites Edukt für den Folgeschritt hergestellt.

### Schritt 6.4

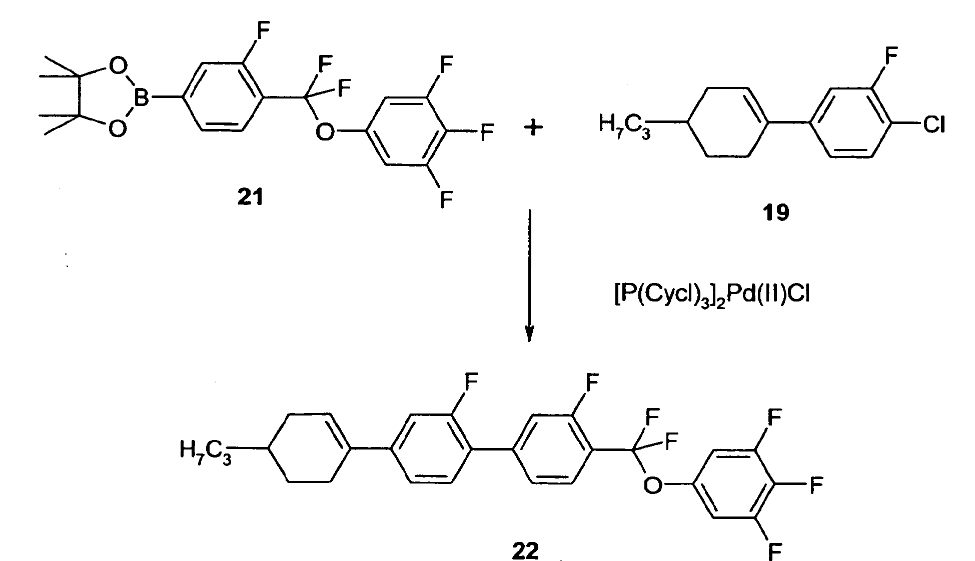

Die Umsetzung und Reinigung erfolgt mit Bis(tricyclohexylphosphin)-palladium(II)chlorid, wässriger Base (Na₂CO₃) im zweiphasigen System Wasser /Toluol wie beschrieben z. B. in der Druckschrift DE 4340490 A1. Die Aufarbeitung und Reinigung erfolgt analog Schritt 5.2.
K 65 SmA (63) N 145 I
Δε 24
Δn 0,190

Analog zu Beispiel 6 werden die folgenden Verbindungen der Formel 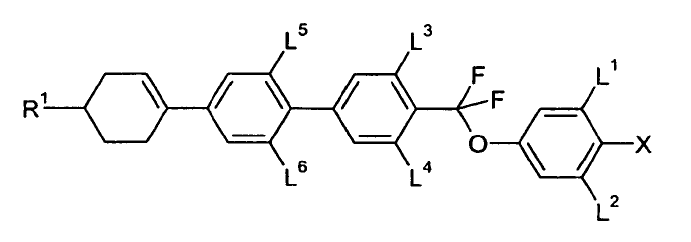 hergestellt:

| R¹ | X | L¹ | L² | L³ | L⁴ | L⁵ | L⁶ | Werte |
|---|---|---|---|---|---|---|---|---|
| H | F | H | H | H | H | H | H | |
| CH₃ | F | H | H | H | H | H | H | |
| C₂H₅ | F | H | H | H | H | H | H | |
| C₃H₇ | F | H | H | H | H | H | H | |
| n-C₄H₉ | F | H | H | H | H | H | H | |
| n-C₅H₁₁ | F | H | H | H | H | H | H | |
| n-C₆H₁₃ | F | H | H | H | H | H | H | |
| H | F | F | H | H | H | H | H | |
| CH₃ | F | F | H | H | H | H | H | |
| C₂H₅ | F | F | H | H | H | H | H | |
| n-C₃H₇ | F | F | H | H | H | H | H | |
| n-C₄H₉ | F | F | H | H | H | H | H | |
| n-C₅H₁₁ | F | F | H | H | H | H | H | |
| n-C₆H₁₃ | F | F | H | H | H | H | H | |
| H | F | F | F | H | H | H | H | |
| CH₃ | F | F | F | H | H | H | H | |
| C₂H₅ | F | F | F | H | H | H | H | |
| n-C₃H₇ | F | F | F | H | H | H | H | K 105 SmE 129 SmC' 134 SmC 150 SmA 169 N 193 I; Δε 19; Δn 0,211 |
| n-C₄H₉ | F | F | F | H | H | H | H | |
| n-C₅H₁₁ | F | F | F | H | H | H | H | |
| n-C₆H₁₃ | F | F | F | H | H | H | H | |
| H | F | H | H | F | H | H | H | |
| CH₃ | F | H | H | F | H | H | H | |
| C₂H₅ | F | H | H | F | H | H | H | |
| C₃H₇ | F | H | H | F | H | H | H | |
| n-C₄H₉ | F | H | H | F | H | H | H | |
| n-C₅H₁₁ | F | H | H | F | H | H | H | |
| n-C₆H₁₃ | F | H | H | F | H | H | H | |
| H | F | F | H | F | H | H | H | |
| CH₃ | F | F | H | F | H | H | H | |
| C₂H₅ | F | F | H | F | H | H | H | |
| n-C₃H₇ | F | F | H | F | H | H | H | |
| n-C₄H₉ | F | F | H | F | H | H | H | |
| n-C₅H₁₁ | F | F | H | F | H | H | H | |
| n-C₆H₁₃ | F | F | H | F | H | H | H | |
| H | F | F | F | F | H | H | H | |
| CH₃ | F | F | F | F | H | H | H | |
| C₂H₅ | F | F | F | F | H | H | H | |
| n-C₃H₇ | F | F | F | F | H | H | H | |
| n-C₄H₉ | F | F | F | F | H | H | H | |
| n-C₅H₁₁ | F | F | F | F | H | H | H | |
| n-C₆H₁₃ | F | F | F | F | H | H | H | |
| H | F | H | H | F | F | H | H | |
| CH₃ | F | H | H | F | F | H | H | |
| C₂H₅ | F | H | H | F | F | H | H | |
| C₃H₇ | F | H | H | F | F | H | H | |
| n-C₄H₉ | F | H | H | F | F | H | H | |
| n-C₅H₁₁ | F | H | H | F | F | H | H | |
| n-C₆H₁₃ | F | H | H | F | F | H | H | |
| H | F | F | H | F | F | H | H | |
| CH₃ | F | F | H | F | F | H | H | |
| C₂H₅ | F | F | H | F | F | H | H | |
| n-C₃H₇ | F | F | H | F | F | H | H | |
| n-C₄H₉ | F | F | H | F | F | H | H | |
| n-C₅H₁₁ | F | F | H | F | F | H | H | |
| n-C₆H₁₃ | F | F | H | F | F | H | H | |
| H | F | F | F | F | F | H | H | |
| CH₃ | F | F | F | F | F | H | H | |
| C₂H₅ | F | F | F | F | F | H | H | |
| n-C₃H₇ | F | F | F | F | F | H | H | K104 N 140 I; Δε 25; Δn 0,197 |
| n-C₄H₉ | F | F | F | F | F | H | H | |
| n-C₅H₁₁ | F | F | F | F | F | H | H | |
| n-C₆H₁₃ | F | F | F | F | F | H | H | |
| H | F | H | H | H | H | F | H | |
| CH₃ | F | H | H | H | H | F | H | |
| C₂H₅ | F | H | H | H | H | F | H | |
| C₃H₇ | F | H | H | H | H | F | H | |
| n-C₄H₉ | F | H | H | H | H | F | H | |
| n-C₅H₁₁ | F | H | H | H | H | F | H | |
| n-C₆H₁₃ | F | H | H | H | H | F | H | |
| H | F | F | H | H | H | F | H | |
| CH₃ | F | F | H | H | H | F | H | |
| C₂H₅ | F | F | H | H | H | F | H | |
| n-C₃H₇ | F | F | H | H | H | F | H | |
| n-C₄H₉ | F | F | H | H | H | F | H | |
| n-C₅H₁₁ | F | F | H | H | H | F | H | |
| n-C₆H₁₃ | F | F | H | H | H | F | H | |
| H | F | F | F | H | H | F | H | |
| CH₃ | F | F | F | H | H | F | H | |
| C₂H₅ | F | F | F | H | H | F | H | |
| n-C₃H₇ | F | F | F | H | H | F | H | K 78 SmA 109 N 161 I; Δε 22; Δn 0,203 |
| n-C₄H₉ | F | F | F | H | H | F | H | |
| n-C₅H₁₁ | F | F | F | H | H | F | H | |
| n-C₆H₁₃ | F | F | F | H | H | F | H | |
| H | F | H | H | F | H | F | H | |
| CH₃ | F | H | H | F | H | F | H | |
| C₂H₅ | F | H | H | F | H | F | H | |
| C₃H₇ | F | H | H | F | H | F | H | |
| n-C₄H₉ | F | H | H | F | H | F | H | |
| n-C₅H₁₁ | F | H | H | F | H | F | H | |
| n-C₆H₁₃ | F | H | H | F | H | F | H | |
| H | F | F | H | F | H | F | H | |
| CH₃ | F | F | H | F | H | F | H | |
| C₂H₅ | F | F | H | F | H | F | H | |
| n-C₃H₇ | F | F | H | F | H | F | H | |
| n-C₄H₉ | F | F | H | F | H | F | H | |
| n-C₅H₁₁ | F | F | H | F | H | F | H | |
| n-C₆H₁₃ | F | F | H | F | H | F | H | |
| H | F | F | F | F | H | F | H | |
| CH₃ | F | F | F | F | H | F | H | |
| C₂H₅ | F | F | F | F | H | F | H | |
| n-C₃H₇ | F | F | F | F | H | F | H | vgl. Beispiel 6 |
| n-C₄H₉ | F | F | F | F | H | F | H | |
| n-C₅H₁₁ | F | F | F | F | H | F | H | |
| n-C₆H₁₃ | F | F | F | F | H | F | H | |
| H | F | H | H | F | F | F | H | |
| CH₃ | F | H | H | F | F | F | H | |
| C₂H₅ | F | H | H | F | F | F | H | |
| C₃H₇ | F | H | H | F | F | F | H | |
| n-C₄H₉ | F | H | H | F | F | F | H | |
| n-C₅H₁₁ | F | H | H | F | F | F | H | |
| n-C₆H₁₃ | F | H | H | F | F | F | H | |
| H | F | F | H | F | F | F | H | |
| CH₃ | F | F | H | F | F | F | H | |
| C₂H₅ | F | F | H | F | F | F | H | |
| n-C₃H₇ | F | F | H | F | F | F | H | |
| n-C₄H₉ | F | F | H | F | F | F | H | |
| n-C₅H₁₁ | F | F | H | F | F | F | H | |
| n-C₆H₁₃ | F | F | H | F | F | F | H | |
| H | F | F | F | F | F | F | H | |
| CH₃ | F | F | F | F | F | F | H | |
| C₂H₅ | F | F | F | F | F | F | H | |
| n-C₃H₇ | F | F | F | F | F | F | H | K 84 N 127 I; Δε 29; Δn 0,183 |
| n-C₄H₉ | F | F | F | F | F | F | H | |
| n-C₅H₁₁ | F | F | F | F | F | F | H | |
| n-C₆H₁₃ | F | F | F | F | F | F | H | |
| H | F | H | H | H | H | F | F | |
| CH₃ | F | H | H | H | H | F | F | |
| C₂H₅ | F | H | H | H | H | F | F | |
| C₃H₇ | F | H | H | H | H | F | F | |
| n-C₄H₉ | F | H | H | H | H | F | F | |
| n-C₅H₁₁ | F | H | H | H | H | F | F | |
| n-C₆H₁₃ | F | H | H | H | H | F | F | |
| H | F | F | H | H | H | F | F | |
| CH₃ | F | F | H | H | H | F | F | |
| C₂H₅ | F | F | H | H | H | F | F | |
| n-C₃H₇ | F | F | H | H | H | F | F | |
| n-C₄H₉ | F | F | H | H | H | F | F | |
| n-C₅H₁₁ | F | F | H | H | H | F | F | |
| n-C₆H₁₃ | F | F | H | H | H | F | F | |
| H | F | F | F | H | H | F | F | |
| CH₃ | F | F | F | H | H | F | F | |
| C₂H₅ | F | F | F | H | H | F | F | |
| n-C₃H₇ | F | F | F | H | H | F | F | K 108 N 140 I; Δε 26; Δn 0,187 |
| n-C₄H₉ | F | F | F | H | H | F | F | |
| n-C₅H₁₁ | F | F | F | H | H | F | F | |
| n-C₆H₁₃ | F | F | F | H | H | F | F | |
| H | F | H | H | F | H | F | F | |
| CH₃ | F | H | H | F | H | F | F | |
| C₂H₅ | F | H | H | F | H | F | F | |
| C₃H₇ | F | H | H | F | H | F | F | |
| n-C₄H₉ | F | H | H | F | H | F | F | |
| n-C₅H₁₁ | F | H | H | F | H | F | F | |
| n-C₆H₁₃ | F | H | H | F | H | F | F | |
| H | F | F | H | F | H | F | F | |
| CH₃ | F | F | H | F | H | F | F | |
| C₂H₅ | F | F | H | F | H | F | F | |
| n-C₃H₇ | F | F | H | F | H | F | F | |
| n-C₄H₉ | F | F | H | F | H | F | F | |
| n-C₅H₁₁ | F | F | H | F | H | F | F | |
| n-C₆H₁₃ | F | F | H | F | H | F | F | |
| H | F | F | F | F | H | F | F | |
| CH₃ | F | F | F | F | H | F | F | |
| C₂H₅ | F | F | F | F | H | F | F | |
| n-C₃H₇ | F | F | F | F | H | F | F | K 83 N 125 I; Δε 29; Δn 0,177 |
| n-C₄H₉ | F | F | F | F | H | F | F | |
| n-C₅H₁₁ | F | F | F | F | H | F | F | |
| n-C₆H₁₃ | F | F | F | F | H | F | F | |
| H | F | H | H | F | F | F | F | |
| H₃ | F | H | H | F | F | F | F | |
| C₂H₅ | F | H | H | F | F | F | F | |
| C₃H₇ | F | H | H | F | F | F | F | |
| n-C₄H₉ | F | H | H | F | F | F | F | |
| n-C₅H₁₁ | F | H | H | F | F | F | F | |
| n-C₆H₁₃ | F | H | H | F | F | F | F | |
| H | F | F | H | F | F | F | F | |
| CH₃ | F | F | H | F | F | F | F | |
| C₂H₅ | F | F | H | F | F | F | F | |
| n-C₃H₇ | F | F | H | F | F | F | F | |
| n-C₄H₉ | F | F | H | F | F | F | F | |
| n-C₅H₁₁ | F | F | H | F | F | F | F | |
| n-C₆H₁₃ | F | F | H | F | F | F | F | |
| H | F | F | F | F | F | F | F | |
| CH₃ | F | F | F | F | F | F | F | |
| C₂H₅ | F | F | F | F | F | F | F | |
| n-C₃H₇ | F | F | F | F | F | F | F | K 84 N 109 I; Δε 33; Δn 0,168 |
| n-C₄H₉ | F | F | F | F | F | F | F | |
| n-C₅H₁₁ | F | F | F | F | F | F | F | |
| n-C₆H₁₃ | F | F | F | F | F | F | F | |
| H | OCF₃ | H | H | H | H | H | H | |
| CH₃ | OCF₃ | H | H | H | H | H | H | |
| C₂H₅ | OCF₃ | H | H | H | H | H | H | |
| C₃H₇ | OCF₃ | H | H | H | H | H | H | |
| n-C₄H₉ | OCF₃ | H | H | H | H | H | H | |
| n-C₅H₁₁ | OCF₃ | H | H | H | H | H | H | |
| n-C₆H₁₃ | OCF₃ | H | H | H | H | H | H | |
| H | OCF₃ | F | H | H | H | H | H | |
| CH₃ | OCF₃ | F | H | H | H | H | H | |
| C₂H₅ | OCF₃ | F | H | H | H | H | H | |
| n-C₃H₇ | OCF₃ | F | H | H | H | H | H | K 31 SmC' 120 SmC 139 SmA' 159 SmA 223 N 228 I; Δε 16; Δn 0,212 |
| n-C₄H₉ | OCF₃ | F | H | H | H | H | H | |
| n-C₅H₁₁ | OCF₃ | F | H | H | H | H | H | |
| n-C₆H₁₃ | OCF₃ | F | H | H | H | H | H | |
| H | OCF₃ | F | F | H | H | H | H | |
| CH₃ | OCF₃ | F | F | H | H | H | H | |
| C₂H₅ | OCF₃ | F | F | H | H | H | H | |
| n-C₃H₇ | OCF₃ | F | F | H | H | H | H | |
| n-C₄H₉ | OCF₃ | F | F | H | H | H | H | |
| n-C₅H₁₁ | OCF₃ | F | F | H | H | H | H | |
| -C₆H₁₃ | OCF₃ | F | F | H | H | H | H | |
| H | OCF₃ | H | H | F | H | H | H | |
| CH₃ | OCF₃ | H | H | F | H | H | H | |
| C₂H₅ | OCF₃ | H | H | F | H | H | H | |
| C₃H₇ | OCF₃ | H | H | F | H | H | H | |
| -C₄H₉ | OCF₃ | H | H | F | H | H | H | |
| n-C₅H₁₁ | OCF₃ | H | H | F | H | H | H | |
| n-C₆H₁₃ | OCF₃ | H | H | F | H | H | H | |
| H | OCF₃ | F | H | F | H | H | H | |
| CH₃ | OCF₃ | F | H | F | H | H | H | |
| C₂H₅ | OCF₃ | F | H | F | H | H | H | |
| n-C₃H₇ | OCF₃ | F | H | F | H | H | H | |
| n-C₄H₉ | OCF₃ | F | H | F | H | H | H | |
| n-C₅H₁₁ | OCF₃ | F | H | F | H | H | H | |
| n-C₆H₁₃ | OCF₃ | F | H | F | H | H | H | |
| H | OCF₃ | F | F | F | H | H | H | |
| CH₃ | OCF₃ | F | F | F | H | H | H | |
| C₂H₅ | OCF₃ | F | F | F | H | H | H | |
| n-C₃H₇ | OCF₃ | F | F | F | H | H | H | |
| n-C₄H₉ | OCF₃ | F | F | F | H | H | H | |
| n-C₅H₁₁ | OCF₃ | F | F | F | H | H | H | |
| n-C₆H₁₃ | OCF₃ | F | F | F | H | H | H | |
| H | OCF₃ | H | H | F | F | H | H | |
| CH₃ | OCF₃ | H | H | F | F | H | H | |
| C₂H₅ | OCF₃ | H | H | F | F | H | H | |
| C₃H₇ | OCF₃ | H | H | F | F | H | H | |
| n-C₄H₉ | OCF₃ | H | H | F | F | H | H | |
| n-C₅H₁₁ | OCF₃ | H | H | F | F | H | H | |
| n-C₆H₁₃ | OCF₃ | H | H | F | F | H | H | |
| H | OCF₃ | F | H | F | F | H | H | |
| CH₃ | OCF₃ | F | H | F | F | H | H | |
| C₂H₅ | OCF₃ | F | H | F | F | H | H | |
| n-C₃H₇ | OCF₃ | F | H | F | F | H | H | |
| n-C₄H₉ | OCF₃ | F | H | F | F | H | H | |
| n-C₅H₁₁ | OCF₃ | F | H | F | F | H | H | |
| n-C₅H₁₃ | OCF₃ | F | H | F | F | H | H | |
| H | OCF₃ | F | F | F | F | H | H | |
| CH₃ | OCF₃ | F | F | F | F | H | H | |
| C₂H₅ | OCF₃ | F | F | F | F | H | H | |
| n-C₃H₇ | OCF₃ | F | F | F | F | H | H | |
| n-C₄H₉ | OCF₃ | F | F | F | F | H | H | |
| n-C₅H₁₁ | OCF₃ | F | F | F | F | H | H | |
| n-C₆H₁₃ | OCF₃ | F | F | F | F | H | H | |
| H | OCF₃ | H | H | H | H | F | H | |
| CH₃ | OCF₃ | H | H | H | H | F | H | |
| C₂H₅ | OCF₃ | H | H | H | H | F | H | |
| C₃H₇ | OCF₃ | H | H | H | H | F | H | |
| n-C₄H₉ | OCF₃ | H | H | H | H | F | H | |
| n-C₅H₁₁ | OCF₃ | H | H | H | H | F | H | |
| n-C₆H₁₃ | OCF₃ | H | H | H | H | F | H | |
| H | OCF₃ | F | H | H | H | F | H | |
| CH₃ | OCF₃ | F | H | H | H | F | H | |
| C₂H₅ | OCF₃ | F | H | H | H | F | H | |
| n-C₃H₇ | OCF₃ | F | H | H | H | F | H | K 31 SmA 177 N 193 1; Δε 18; Δn 0,197 |
| n-C₄H₉ | OCF₃ | F | H | H | H | F | H | |
| n-C₅H₁₁ | OCF₃ | F | H | H | H | F | H | |
| n-C₆H₁₃ | OCF₃ | F | H | H | H | F | H | |
| H | OCF₃ | F | F | H | H | F | H | |
| CH₃ | OCF₃ | F | F | H | H | F | H | |
| C₂H₅ | OCF₃ | F | F | H | H | F | H | |
| n-C₃H₇ | OCF₃ | F | F | H | H | F | H | |
| n-C₄H₉ | OCF₃ | F | F | H | H | F | H | |
| n-C₅H₁₁ | OCF₃ | F | F | H | H | F | H | |
| n-C₆H₁₃ | OCF₃ | F | F | H | H | F | H | |
| H | OCF₃ | H | H | F | H | F | H | |
| CH₃ | OCF₃ | H | H | F | H | F | H | |
| C₂H₅ | OCF₃ | H | H | F | H | F | H | |
| C₃H₇ | OCF₃ | H | H | F | H | F | H | |
| n-C₄H₉ | OCF₃ | H | H | F | H | F | H | |
| n-C₅H₁₁ | OCF₃ | H | H | F | H | F | H | |
| n-C₆H₁₃ | OCF₃ | H | H | F | H | F | H | |
| H | OCF₃ | F | H | F | H | F | H | |
| CH₃ | OCF₃ | F | H | F | H | F | H | |
| C₂H₅ | OCF₃ | F | H | F | H | F | H | |
| n-C₃H₇ | OCF₃ | F | H | F | H | F | H | |
| n-C₄H₉ | OCF₃ | F | H | F | H | F | H | |
| n-C₅H₁₁ | OCF₃ | F | H | F | H | F | H | |
| n-C₆H₁₃ | OCF₃ | F | H | F | H | F | H | |
| H | OCF₃ | F | F | F | H | F | H | |
| CH₃ | OCF₃ | F | F | F | H | F | H | |
| C₂H₅ | OCF₃ | F | F | F | H | F | H | |
| n-C₃H₇ | OCF₃ | F | F | F | H | F | H | |
| n-C₄H₉ | OCF₃ | F | F | F | H | F | H | |
| n-C₅H₁₁ | OCF₃ | F | F | F | H | F | H | |
| n-C₆H₁₃ | OCF₃ | F | F | F | H | F | H | |
| H | OCF₃ | H | H | F | F | F | H | |
| CH₃ | OCF₃ | H | H | F | F | F | H | |
| C₂H₅ | OCF₃ | H | H | F | F | F | H | |
| C₃H₇ | OCF₃ | H | H | F | F | F | H | |
| n-C₄H₉ | OCF₃ | H | H | F | F | F | H | |
| n-C₅H₁₁ | OCF₃ | H | H | F | F | F | H | |
| n-C₆H₁₃ | OCF₃ | H | H | F | F | F | H | |
| H | OCF₃ | F | H | F | F | F | H | |
| CH₃ | OCF₃ | F | H | F | F | F | H | |
| C₂H₅ | OCF₃ | F | H | F | F | F | H | |
| n-C₃H₇ | OCF₃ | F | H | F | F | F | H | K 57 SmA 125 N 153 1; Δε 25; Δn 0,183 |
| n-C₄H₉ | OCF₃ | F | H | F | F | F | H | |
| n-C₅H₁₁ | OCF₃ | F | H | F | F | F | H | |
| n-C₆H₁₃ | OCF₃ | F | H | F | F | F | H | |
| H | OCF₃ | F | F | F | F | F | H | |
| CH₃ | OCF₃ | F | F | F | F | F | H | |
| C₂H₅ | OCF₃ | F | F | F | F | F | H | |
| n-C₃H₇ | OCF₃ | F | F | F | F | F | H | |
| n-C₄H₉ | OCF₃ | F | F | F | F | F | H | |
| n-C₅H₁₁ | OCF₃ | F | F | F | F | F | H | |
| n-C₆H₁₃ | OCF₃ | F | F | F | F | F | H | |
| H | OCF₃ | H | H | H | H | F | F | |
| CH₃ | OCF₃ | H | H | H | H | F | F | |
| C₂H₅ | OCF₃ | H | H | H | H | F | F | |
| C₃H₇ | OCF₃ | H | H | H | H | F | F | |
| n-C₄H₉ | OCF₃ | H | H | H | H | F | F | |
| n-C₅H₁₁ | OCF₃ | H | H | H | H | F | F | |
| n-C₆H₁₃ | OCF₃ | H | H | H | H | F | F | |
| H | OCF₃ | F | H | H | H | F | F | |
| CH₃ | OCF₃ | F | H | H | H | F | F | |
| C₂H₅ | OCF₃ | F | H | H | H | F | F | |
| n-C₃H₇ | OCF₃ | F | H | H | H | F | F | |
| n-C₄H₉ | OCF₃ | F | H | H | H | F | F | |
| n-C₅H₁₁ | OCF₃ | F | H | H | H | F | F | |
| n-C₆H₁₃ | OCF₃ | F | H | H | H | F | F | |
| H | OCF₃ | F | F | H | H | F | F | |
| CH₃ | OCF₃ | F | F | H | H | F | F | |
| C₂H₅ | OCF₃ | F | F | H | H | F | F | |
| n-C₃H₇ | OCF₃ | F | F | H | H | F | F | |
| n-C₄H₉ | OCF₃ | F | F | H | H | F | F | |
| n-C₅H₁₁ | OCF₃ | F | F | H | H | F | F | |
| n-C₆H₁₃ | OCF₃ | F | F | H | H | F | F | |
| H | OCF₃ | H | H | F | H | F | F | |
| CH₃ | OCF₃ | H | H | F | H | F | F | |
| C₂H₅ | OCF₃ | H | H | F | H | F | F | |
| C₃H₇ | OCF₃ | H | H | F | H | F | F | |
| n-C₄H₉ | OCF₃ | H | H | F | H | F | F | |
| n-C₅H₁₁ | OCF₃ | H | H | F | H | F | F | |
| n-C₆H₁₃ | OCF₃ | H | H | F | H | F | F | |
| H | OCF₃ | F | H | F | H | F | F | |
| CH₃ | OCF₃ | F | H | F | H | F | F | |
| C₂H₅ | OCF₃ | F | H | F | H | F | F | |
| n-C₃H₇ | OCF₃ | F | H | F | H | F | F | |
| n-C₄H₉ | OCF₃ | F | H | F | H | F | F | |
| n-C₅H₁₁ | OCF₃ | F | H | F | H | F | F | |
| n-C₆H₁₃ | OCF₃ | F | H | F | H | F | F | |
| H | OCF₃ | F | F | F | H | F | F | |
| CH₃ | OCF₃ | F | F | F | H | F | F | |
| C₂H₅ | OCF₃ | F | F | F | H | F | F | |
| n-C₃H₇ | OCF₃ | F | F | F | H | F | F | |
| n-C₄H₉ | OCF₃ | F | F | F | H | F | F | |
| n-C₅H₁₁ | OCF₃ | F | F | F | H | F | F | |
| n-C₆H₁₃ | OCF₃ | F | F | F | H | F | F | |
| H | OCF₃ | H | H | F | F | F | F | |
| CH₃ | OCF₃ | H | H | F | F | F | F | |
| C₂H₅ | OCF₃ | H | H | F | F | F | F | |
| C₃H₇ | OCHF₃ | H | H | F | F | F | F | |
| n-C₄H₉ | OCF₃ | H | H | F | F | F | F | |
| n-C₅H₁₁ | OCF₃ | H | H | F | F | F | F | |
| n-C₆H₁₃ | OCF₃ | H | H | F | F | F | F | |
| H | OCF₃ | F | H | F | F | F | F | |
| CH₃ | OCF₃ | F | H | F | F | F | F | |
| C₂H₅ | OCF₃ | F | H | F | F | F | F | |
| n-C₃H₇ | OCF₃ | F | H | F | F | F | F | |
| n-C₄H₉ | OCF₃ | F | H | F | F | F | F | |
| n-C₅H₁₁ | OCF₃ | F | H | F | F | F | F | |
| n-C₆H₁₃ | OCF₃ | F | H | F | F | F | F | |
| H | OCF₃ | F | F | F | F | F | F | |
| CH₃ | OCF₃ | F | F | F | F | F | F | |
| C₂H₅ | OCF₃ | F | F | F | F | F | F | |
| n-C₃H₇ | OCF₃ | F | F | F | F | F | F | |
| n-C₄H₉ | OCF₃ | F | F | F | F | F | F | |
| n-C₅H₁₁ | OCF₃ | F | F | F | F | F | F | |
| n-C₆H₁₃ | OCF₃ | F | F | F | F | F | F | |

### Beispiel 7

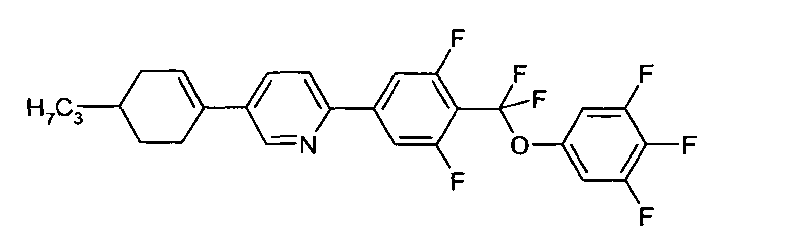

### Schritt 7.1

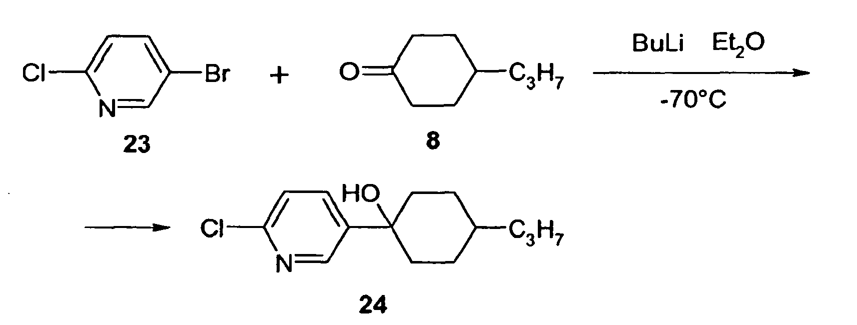

Eine Lösung des Pyridins (50,0 g ; 260 mmol) in 400 ml Diethylether wird unter Stickstoff bei -70 °C mit 180 ml (290 mmol) 15%igem BuLi in n-Hexan versetzt. Nach 60 min wird ebenfalls bei tiefer Temperatur eine Lösung von 36,4 g (260 mmol) des Cyclohexylketons in 200 ml Diethylether in den Ansatz gegeben. Nach einer weiteren Stunde wird der Ansatz auf -20 °C erwärmt, auf Eiswasser gegeben. Die organische Phase wird über Natriumsulfat getrocknet und eingeengt. Der erhaltene Rückstand wird ohne weitere Reinigung in der Folgestufe eingesetzt.

### Schritt 7.2

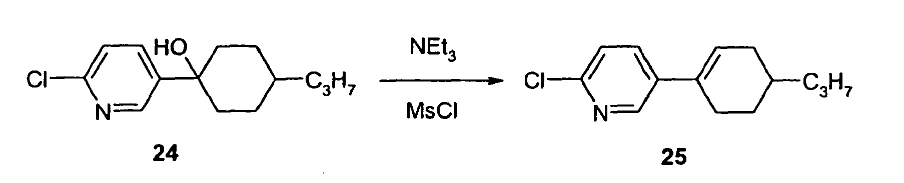

Unter Stickstoff werden 66 g (260,0 mmol) des Alkoholderivats in 800 ml Dichlormethan und 108 ml Triethylamin gelöst und bei 0 °C mit 26,2 ml (340 mmol) Methansulfonsäurechlorid (MsCI) versetzt. Der Ansatz wird über Nacht bei RT gerührt. Anschließend wird das Reaktionsgemisch auf Wasser gegeben und mit MTB-Ether extrahiert. Die organische Phase wird eingeengt, und der erhaltene Rückstand über Kieselgel gegeben (MTB-Ether/n-Heptan 1:4). Der Rückstand wird ohne weitere Reinigung in der folgenden Stufe eingesetzt.

### Schritt 7.3

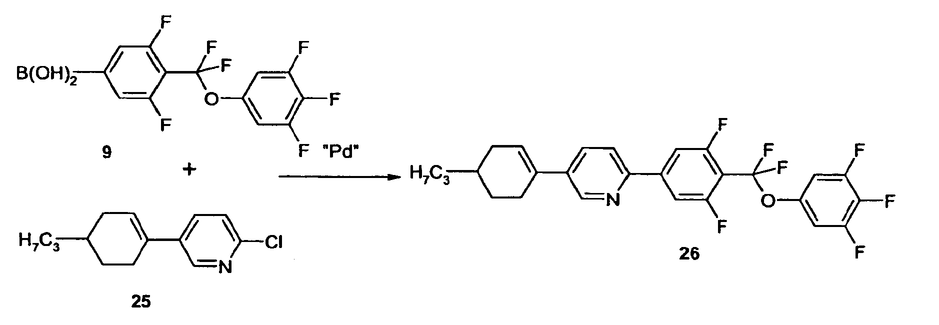

8,7 g (30 mmol) Natriummetaborat-Octahydrat werden in 15 ml Wasser vorgelegt und mit 40 ml THF, 0,10 ml Hydraziniumhydroxid und 300 mg Bis(triphenylphosphin)-palladium(II)chlorid versetzt und 5 min bei RT gerührt. Anschließend wird eine Lösung von 21,5 g (35%ig; 20 mmol) des Boronsäure-Derivats und 4,7 g (20 mmol) des Pyridinchlorids in den Ansatz gegeben. Nach 15 h unter Rückfluss wird das Reaktionsgemisch mit MTB-Ether extrahiert. Die organische Phase wird eingeengt. Der Rückstand wird über Kieselgel (n-Heptan) filtriert. Die Endreinigung des Produktes erfolgt durch Kristallisation aus Heptan.
K 73 SmA (73) N 138 I
Δε 30
in 0,197

Analog wird hergestellt:

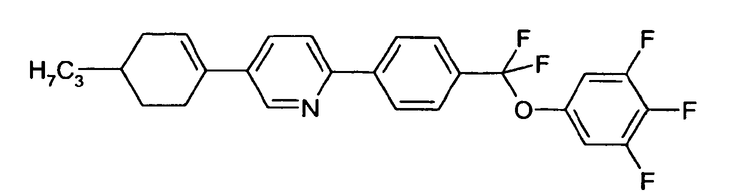

K 100 SmC 107 SmA 184 N 195 I
Δε 24
Δn 0,215

### Beispiel 8

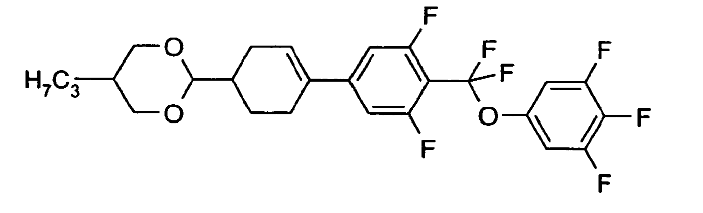

Analog zu den Verbindungen aus Beispiel 2 werden die entsprechenden Dioxanverbindungen hergestellt.
K 84 N 121 I
Δε 34
In 0,123.

Weitere Kombinationen der Ausführungsformen und Varianten der Erfindung gemäß der Beschreibung ergeben sich aus den folgenden Ansprüchen.

## Patentansprüche

1. Verbindungen der Formel I, 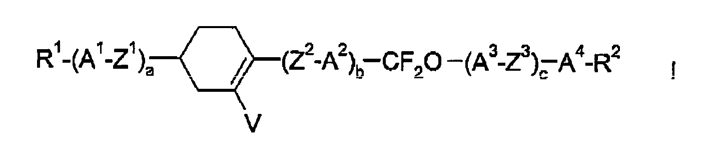 worin
R¹ jeweils unabhängig voneinander H, F, CI, Br, einen halogenierten oder unsubstituierten Alkylrest mit 1 bis 15 C-Atomen, wobei in diesen Resten auch eine oder mehrere CH₂-Gruppen jeweils unabhängig voneinander durch -C≡C-, -CH=CH-, -(CO)O., -O(CO)-, -(CO)- oder -O- so ersetzt sein können, dass O-Atome nicht direkt miteinander verknüpft sind, wobei R¹ auch CN, SCN, NCS oder SF₅ bedeuten kann,
mit der Maßgabe, dass R¹ keine Estergruppe der Formel -(CO)O-C₂H₅ ist.
R² einen Rest X der Bedeutung F, CI, OCF₃, OCHF₂, OCHFCF₃, OCF₂CHFCF₃, CF₃, CN, SF₅ oder NCS,
A¹ und A² j eweils unabhängig voneinander, gleich oder verschieden:
a) trans-1,4-Cyclohexylen oder Cyclohexenylen, worin auch eine oder mehrere nicht benachbarte CH₂-Gruppen durch -O- und/oder -S- ersetzt sein können und worin H durch F substituiert sein kann,
b) 1,4-phenylen, worin eine oder zwei CH-Gruppen durch N ersetzt sein können und worin auch ein oder mehrere H-Atome gegen Br, CI, F, CN, Methyl, Methoxy oder eine ein- oder mehrfach fluorierte Methyl- oder Methoxygruppe ersetzt sein können,
oder
c) ein Rest aus der Gruppe 1,4-Bicyclo(2,2,2)-octylen, Piperidin-1,4-diyl, Cylcobut-1,3-diyl, Spiro[3.3]heptan-2,6-diyl, 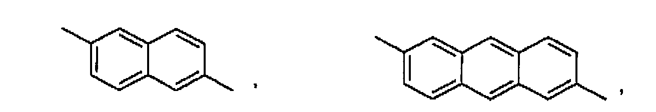 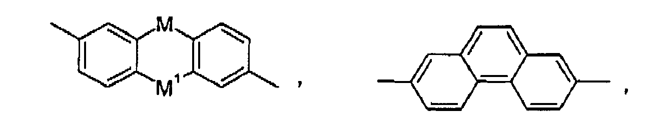 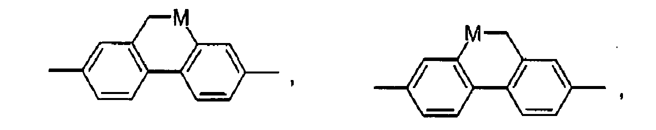 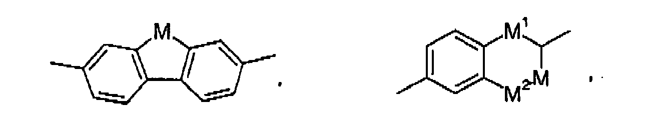 worin Wasserstoffatome ein oder mehrfach durch F, CN, SCN, SF₅, CH₂F, CHF₂, CF₃, OCH₂F, OCHF₂ oder OCF₃ substituiert sein können,
eine oder mehrere Doppelbindungen durch Einfachbindungen ersetzt sein können, M, M¹ oder M² -O-, -S-, -CH₂, -CHY- oder -CYY¹-, so dass benachbarte Gruppen nicht gleichzeitig -O- oder -S-bedeuten, und
Y und Y¹ Cl, F, CN, OCF₃ oder CF₃ bedeuten,
A³ und A⁴ jeweils unabhängig voneinander, gleich oder verschieden:
b) 1,4-Phenylen, worin eine oder zwei CH-Gruppen durch N ersetzt sein können und worin auch ein oder mehrere H-Atome gegen Br, CI, F, CN, Methyl, Methoxy oder eine ein- oder mehrfach fluorierte Methyl- oder Methoxygruppe ersetzt sein können,
oder
c) ein Rest aus der Gruppe 1,4-Bicyclo(2,2,2)-octylen, Piperidin-1 ,4-diyl, Cylcobut-1,3-diyl, Spiro[3.3]heptan-2,6-diyl, 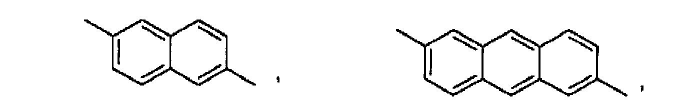 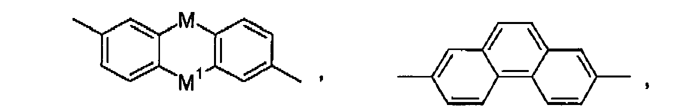 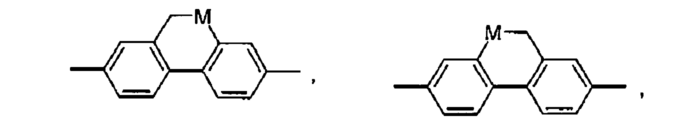 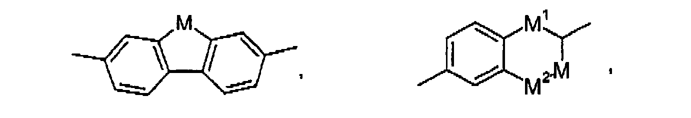 worin Wasserstoffatome ein oder mehrfach durch F, CN, SCN, SF₅, CH₂F, CHF₂, CF₃, OCH₂F, OCHF₂ oder OCF₃ substituiert sein können,
eine oder mehrere Doppelbindungen durch Einfachbindungen ersetzt sein können, M, M¹ oder M² -O-, -S-, -CH₂-, -CHY- oder -CYY¹-, so dass benachbarte Gruppen nicht gleichzeitig -O- oder -S-bedeuten, und
Y und Y¹ CI, F, CN, OCF₃ oder CF₃ bedeuten,
V H oder F,
Z¹, Z² und Z³ jeweils unabhängig voneinander, gleich oder verschieden,
eine Einfachbindung, -CH₂O-, -(CO)O-, -CF₂O-, -CH₂CH₂CF₂O-, -CF₂CF₂-, -CH₂CF₂-, -CH₂CH₂-, -(CH₂)₄-,
-CH=CH-, -CH=CF-, -CF=CF- oder-C≡C-, wobei asymmetrische Brücken nach beiden Seiten orientiert sein können, und
a 0, 1 oder 2,
b 1 oder 2, und
c 0, 1 oder 2
wobei a + b + ≤ 4 ist,
bedeuten.

2. Verbindungen nach Anspruch 1 der Formel IA 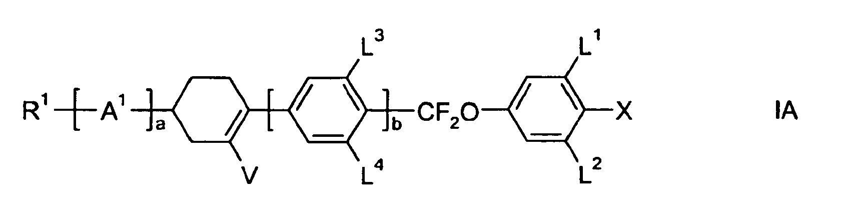 worin
R¹, A¹, a, b und V die in Anspruch 1 für Formel I angegebenen Bedeutungen haben,
X F, OCF₃, CN, CF₃, SCN, SF₅, NCS, Cl, OCHF₂, OCHFCF₃, OCF₂CHFCF₃,
V H oder F, und
L¹, L², L³ und L⁴ jeweils unabhängig voneinander H oder F,
bedeuten.

3. Verbindungen nach Anspruch 1 oder 2, **dadurch gekennzeichnet, dass**
R¹ Alkyl, Alkoxy, Alkenyl oder Alkenyloxy mit bis zu 8 Kohlenstoffatomen bedeutet.

4. Verbindungen nach einem oder mehreren der Ansprüche 1 bis 3, **dadurch gekennzeichnet, dass** L¹ Fluor und L² Fluor oder Wasserstoff bedeuten.

5. Verbindungen nach einem oder mehreren der Ansprüche 1 bis 4 der Formeln 11 bis 15, 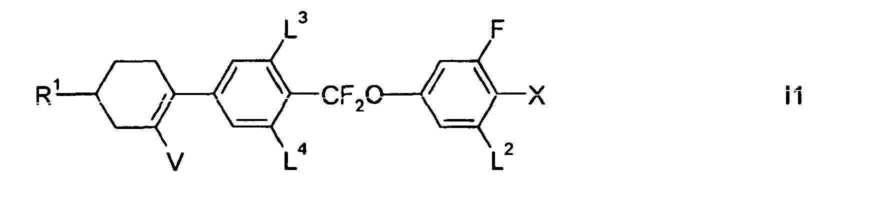 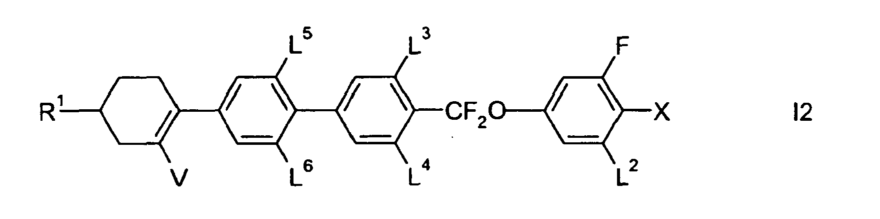 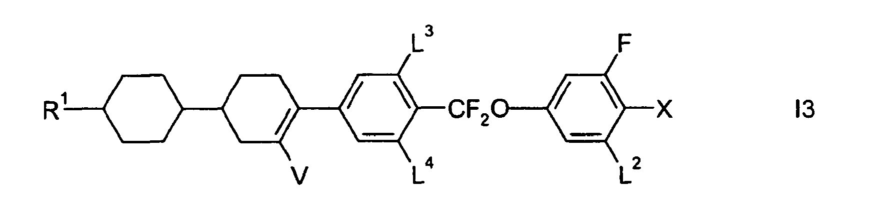 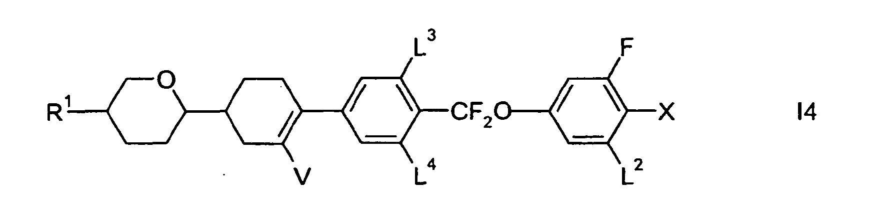 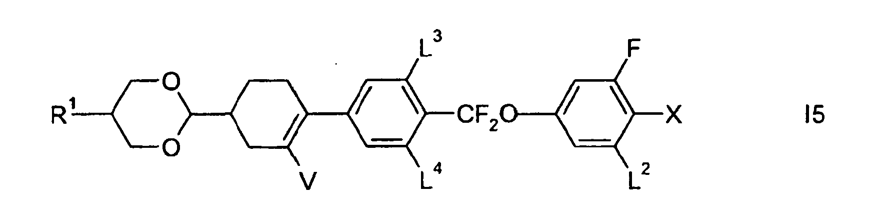 worin R¹und V die in Anspruch 1 angegebenen Bedeutungen haben und
X F, OCF₃, CN, CF₃, SCN, SF₅. NCS, Cl, OCHF₂, OCHFCF₃, OCF₂CHFCF₃ und
L², L³, L⁴, L⁵ und L⁶ H oder F
bedeuten.

6. Verbindungen nach einem oder mehreren der Ansprüche 1 bis 5, **dadurch gekennzeichnet, dass** L¹ und L² Fluor bedeuten.

7. Verbindungen nach einem oder mehreren der Ansprüche 1 bis 6, **dadurch gekennzeichnet, dass** V Wasserstoff bedeutet.

8. Verbindungen nach einem oder mehreren der Ansprüche 1 bis 6, **dadurch gekennzeichnet, dass** V Fluor bedeutet.

9. Verfahren zur Herstellung von Verbindungen der Formel I nach einem oder mehreren der Ansprüche 1 bis 7, worin V Wasserstoff bedeutet umfassend einen Verfahrensschritt, **dadurch gekennzeichnet, dass** ein Cyclohexanketon der Formel 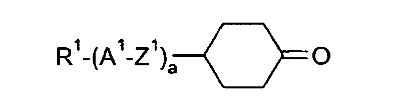 worin R¹, A¹, Z¹ und a wie in Anspruch 1 definiert sind,
an der Carbonylgruppe mit einer Magnesium-organischen Verbindung der Formel
MgHal-(Z²-A²)_{b}-CF₂O-(A³-Z³)_{c}-A⁴-R²
worin Z², Z³, A², A³, A⁴, a, c und R² wie in Anspruch 1 definiert sind und
Hal Cl oder Br bedeutet,
zur Reaktion gebracht wird.

10. Verfahren zur Herstellung von Verbindungen der Formel 1 nach einem oder mehreren der Ansprüche 1 bis 8, worin V Fluor oder Wasserstoff bedeutet umfassend einen Verfahrensschritt, **dadurch gekennzeichnet, dass** ein Cyclohexen der Formel 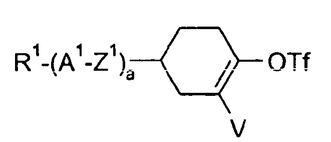 worin R¹, A¹, Z¹ und a wie in Anspruch 1 definiert sind,
mit einer Boronsäure oder einem offenkettigen oder cyclischen Boronsäuresäureester der Formeln
Z`b CF2O-'As_Z3`C Aa_R2 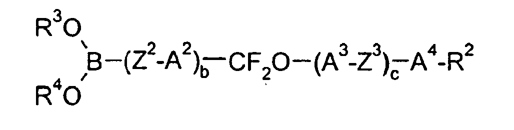
worin Z², Z³, A², A³, A⁴, a, b und R² wie in Anspruch 1 definiert sind, und
R³, R⁴ Alkyl mit 1-12 C-Atomen oder R³+R⁴ zusammen auch ein
Alkylen, insbesondere der Formeln
-CH₂-(CH₂)ₚ-CH₂- und -C(CH₃)₂C(CH₃)₂-,
oder 1,2-Phenylen bedeuten,
wobei R¹, R² und R¹+R² auch substituiert sein können und
wobei p 0 oder 1 ist,
in Gegenwart eines Übergangsmetallkatalysators zur Reaktion gebracht wird.

11. Verwendung einer oder mehrerer Verbindungen der Formel 1 nach einem oder mehreren der Ansprüche 1 bis 8 als Komponenten in einem flüssigkristallinen Medium.

12. Flüssigkristallines Medium enthaltend mindestens zwei mesogene Verbindungen, **dadurch gekennzeichnet, dass** es mindestens eine Verbindung der Formel I nach einem oder mehreren der Ansprüche 1 bis 8 enthält.

13. Verwendung des flüssigkristallinen Mediums nach Anspruch 12 für elektrooptische Zwecke.

14. Elektrooptische Flüssigkristallanzeige enthaltend ein flüssigkristallines Medium nach Anspruch 12.

## Claims

1. Compounds of the formula I, 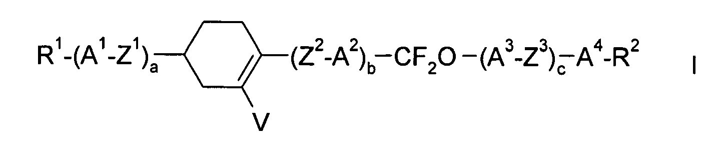 in which
R¹ in each case, independently of one another, denotes H, F, Cl, Br, a halogenated or unsubstituted alkyl radical having 1 to 15 C atoms, where one or more CH₂ groups in these radicals may also each be replaced, independently of one another, by -C≡C-, -CH=CH-, -(CO)O-, -O(CO)-, -(CO)- or -O- in such a way that O atoms are not linked directly to one another, where R¹ may also denote CN, SCN, NCS or SF₅,
with the proviso that R¹ is not an ester group of the formula -(CO)O-C₂H₅,
R² denotes a radical X having the meaning F, CI, OCF₃, OCHF₂, OCHFCF₃, OCF₂CHFCF₃, CF₃, CN, SF₅ or NCS,
A¹ and A² each, independently of one another, identically or differently, denote:
a) trans-1,4-cyclohexylene or cyclohexenylene, in which, in addition, one or more non-adjacent CH₂ groups may be replaced by -O- and/or -S- and in which H may be substituted by F,
b) 1,4-phenylene, in which one or two CH groups may be replaced by N and in which, in addition, one or more H atoms may be replaced by Br, Cl, F, CN, methyl, methoxy or a mono- or polyfluorinated methyl or methoxy group,
or
c) a radical from the group 1,4-bicyclo[2.2.2]octylene, piperidine-1,4-diyl, cyclobutane-1,3-diyl, spiro[3.3]heptane-2,6-diyl, 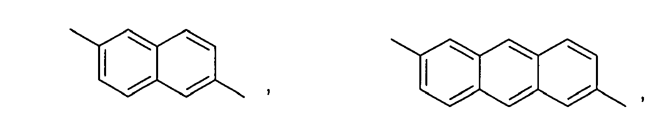 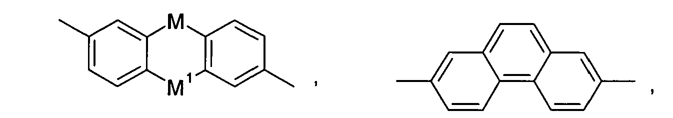 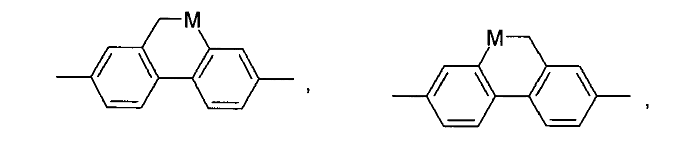 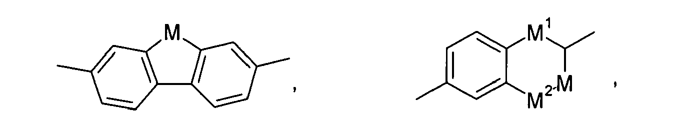 in which one or more hydrogen atoms may be substituted by F, CN, SCN, SF₅, CH₂F, CHF₂, CF₃, OCH₂F, OCHF₂ or OCF₃,
one or more double bonds may be replaced by single bonds,
M, M¹ or M² denotes -O-, -S-, -CH₂-, -CHY- or -CYY¹- in such a way that adjacent groups do not simultaneously denote -O- or -S-, and
Y and Y¹ denote Cl, F, CN, OCF₃ or CF₃,
A³ and A⁴ each, independently of one another, identically or differently, denote:
b) 1,4-phenylene, in which one or two CH groups may be replaced by N and in which, in addition, one or more H atoms may be replaced by Br, CI, F, CN, methyl, methoxy or a mono- or polyfluorinated methyl or methoxy group,
or
c) a radical from the group 1,4-bicyclo[2.2.2]octylene, piperidine-1,4-diyl, cyclobutane-1,3-diyl, spiro[3.3]heptane-2,6-diyl, 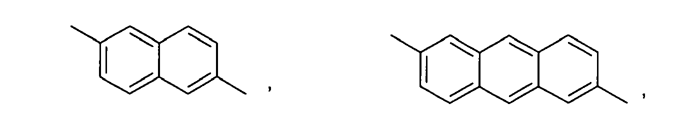 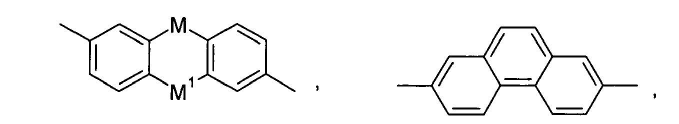 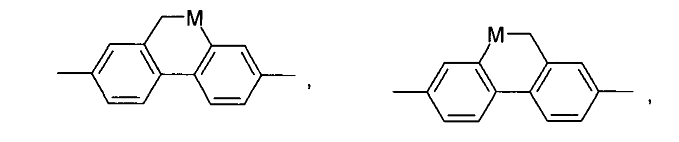 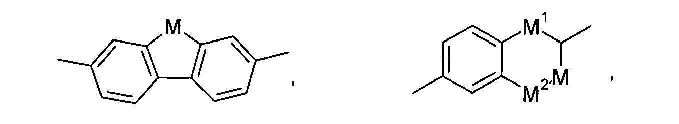 in which one or more hydrogen atoms may be substituted by F, CN, SCN, SF₅, CH₂F, CHF₂, CF₃, OCH₂F, OCHF₂ or OCF₃,
one or more double bonds may be replaced by single bonds,
M, M¹ or M² denotes -O-, -S-, -CH₂-, -CHY- or -CYY¹- in such a way that adjacent groups do not simultaneously denote -O- or -S-, and
Y and Y¹ denote Cl, F, CN, OCF₃ or CF₃,
V denotes H or F,
Z¹, Z² and Z³ each, independently of one another, identically or differently, denote
a single bond, -CH₂O-, -(CO)O-, -CF₂O-, -CH₂CH₂CF₂O-, -CF₂CF₂-, -CH₂CF₂-, -CH₂CH₂-, -(CH₂)₄-, -CH=CH-, -CH=CF-, -CF=CF- or -C≡C-, where asymmetrical bridges may be oriented to both sides, and
a denotes 0, 1 or 2,
b denotes 1 or 2, and
c denotes 0, 1 or 2,
where a + b + c is ≤ 4.

2. Compounds according to Claim 1 of the formula IA 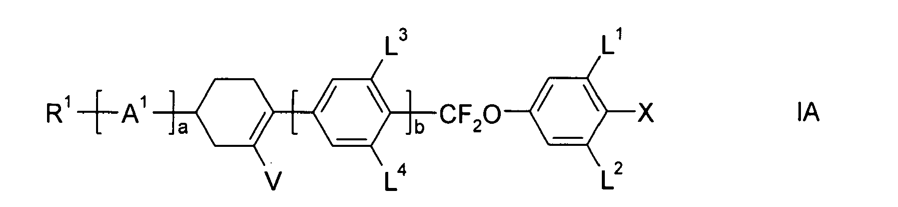 in which
R¹, A¹, a, b and V have the meanings indicated for formula I in Claim 1,
X denotes F, OCF₃, CN, CF₃, SCN, SF₅, NCS, Cl, OCHF₂, OCHFCF₃, OCF₂CHFCF₃,
V denotes H or F, and
L¹, L², L³ and L⁴ each, independently of one another, denote H or F.

3. Compounds according to Claim 1 or 2, **characterised in that**
R¹ denotes alkyl, alkoxy, alkenyl or alkenyloxy having up to 8 carbon atoms.

4. Compounds according to one or more of Claims 1 to 3, **characterised in that** L¹ denotes fluorine and L² denotes fluorine or hydrogen.

5. Compounds according to one or more of Claims 1 to 4 of the formulae 11 to 15, 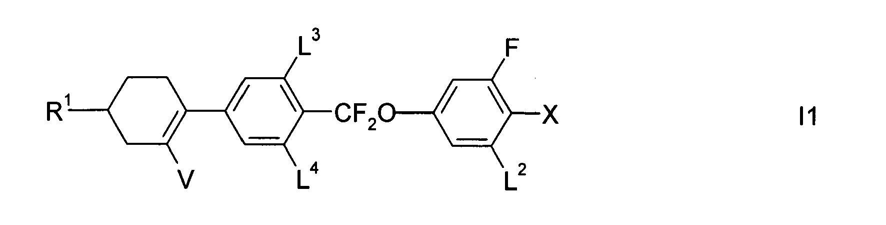 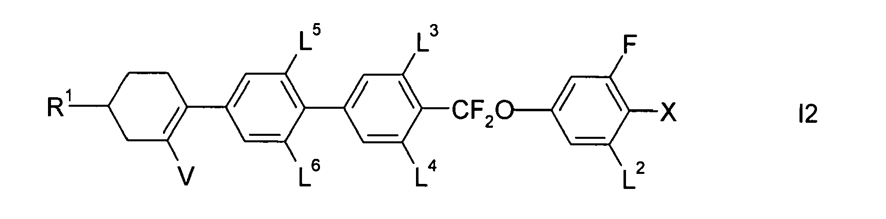 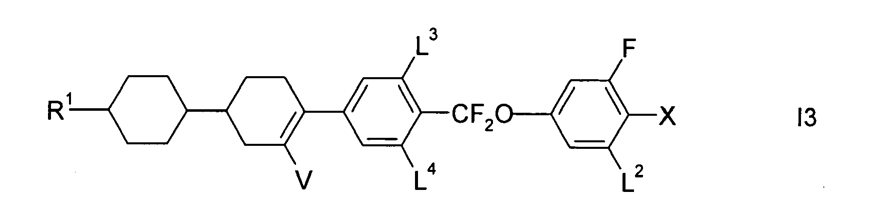 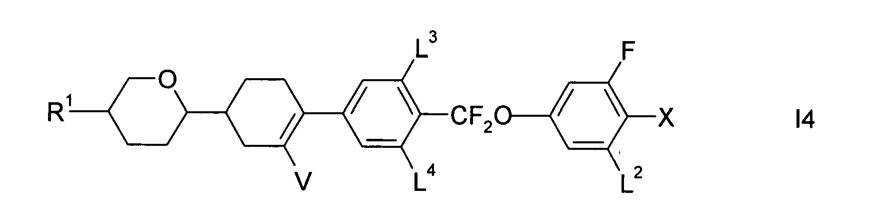 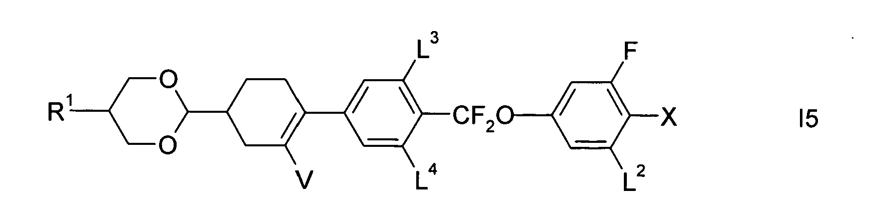 in which R¹ and V have the meanings indicated in Claim 1, and
X denotes F, OCF₃, CN, CF₃, SCN, SF₅, NCS, CI, OCHF₂, OCHFCF₃, OCF₂CHFCF₃, and
L², L³, L¹, L⁵ and L⁶ denote H or F.

6. Compounds according to one or more of Claims 1 to 5, **characterised in that** L¹ and L² denote fluorine.

7. Compounds according to one or more of Claims 1 to 6, **characterised in that** V denotes hydrogen.

8. Compounds according to one or more of Claims 1 to 6, **characterised in that** V denotes fluorine.

9. Process for the preparation of compounds of the formula I according to one or more of Claims 1 to 7 in which V denotes hydrogen comprising a process step, **characterised in that** a cyclohexane ketone of the formula 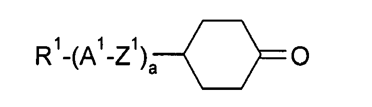 in which R¹, A¹, Z¹ and a are as defined in Claim 1,
is reacted at the carbonyl group with an organomagnesium compound of the formula
MgHal-(Z²-A²)_{b}-CF₂O-(A³-Z³)A⁴-R²
in which Z², Z³, A², A³, A⁴, a, c and R² are as defined in Claim 1, and
Hal denotes Cl or Br.

10. Process for the preparation of compounds of the formula I according to one or more of Claims 1 to 8 in which V denotes fluorine or hydrogen comprising a process step, **characterised in that** a cyclohexene of the formula 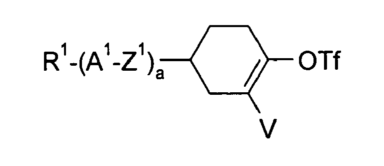 in which R¹, A¹, Z¹ and a are as defined in Claim 1,
is reacted with a boronic acid or an open-chain or cyclic boronic acid ester of the formulae
(OH)₂B-(Z²-A²)_{b}-CF₂O-(A³-Z³)_{c}-A⁴-R² 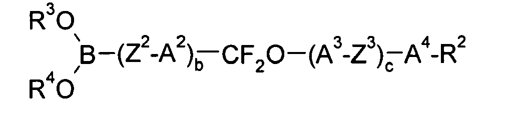
in which Z², Z³, A², A³, A⁴, a, b and R² are as defined in Claim 1, and
R³, R⁴ denote alkyl having 1-12 C atoms or R³+R⁴ together also denote an alkylene, in particular of the formulae
-CH₂-(CH₂)ₚ-CH₂- and -C(CH₃)₂C(CH₃)₂-,
or 1,2-phenylene,
where R¹, R² and R¹+R² may also be substituted and where p is 0 or 1,
in the presence of a transition-metal catalyst.

11. Use of one or more compounds of the formula I according to one or more of Claims 1 to 8 as components in a liquid-crystalline medium.

12. Liquid-crystalline medium comprising at least two mesogenic compounds, **characterised in that** it comprises at least one compound of the formula I according to one or more of Claims 1 to 8.

13. Use of the liquid-crystalline medium according to Claim 12 for electro-optical purposes.

14. Electro-optical liquid-crystal display containing a liquid-crystalline medium according to Claim 12.

## Revendications

1. Composés de la formule 1, 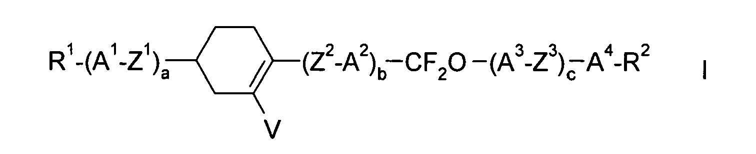 dans laquelle
R¹ représente, dans chaque cas indépendamment des autres, H, F, Cl, Br, un radical alkyle halogéné ou non substitué comportant de 1 à 15 atomes de C, où un ou plusieurs groupes CH₂ dans ces radicaux peuvent également être chacun remplacés, indépendamment les uns des autres, par -C---C-, -CH=CH-, -(CO)O-, -O(CO)-, -(CO)- ou -O- de telle sorte que des atomes de O ne soient pas liés directement les uns aux autres, où R¹ peut également représenter CN, SCN, NCS ou SF₅,
sous réserve que R¹ n'est pas un groupe ester de la formule -(CO)O-C₂H₅,
R² représente un radical X présentant la signification F, Cl, OCF₃, OCHF₂, OCHFCF₃, OCF₂CHFCF₃, CF₃, CN, SF₅ ou NCS,
A¹ et A² représentent, chacun indépendamment de l'autre, de manière identique ou différente :
a) trans-1,4-cyclohexylène ou cyclohexénylène, où, en addition, un ou plusieurs groupes CH₂ non adjacents peuvent être remplacés par -O- et/ou -S- et où H peut être substitué par F,
b) 1,4-phénylène, où un ou deux groupes CH peuvent être remplacés par N et où, en addition, un ou plusieurs atomes de H peuvent être remplacés par Br, CI, F, CN, méthyle, méthoxy ou un groupe méthyle ou méthoxy mono- ou polyfluoré,
ou
c) un radical pris parmi le groupe 1,4-bicyclo[2.2.2]octylène, pipéridine-1,4-diyle, cyclobutane-1,3-diyle, spiro-[3.3]heptane-2,6-diyle, 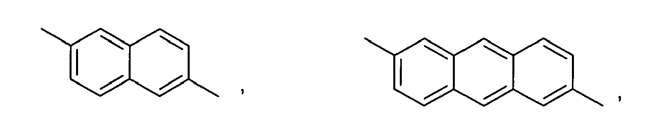 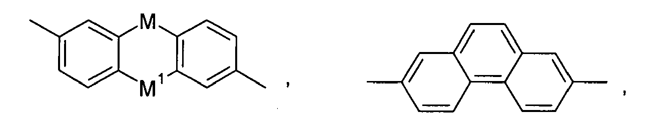 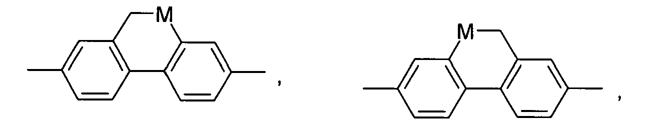 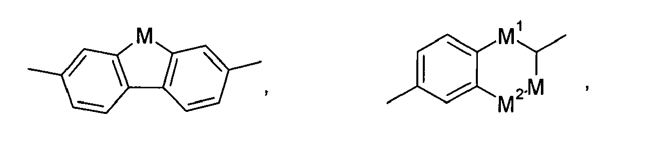 où un ou plusieurs atomes d'hydrogène peuvent être substitués par F, CN, SCN, SF₅, CH₂F, CHF₂, CF₃, OCH₂F, OCHF₂ ou OCF₃,
une ou plusieurs liaisons doubles peuvent être remplacées par des liaisons simples,
M, M¹ ou M² représente -O-, -S-, -CH₂-, -CHY- ou -CYY¹- de telle sorte que des groupes adjacents ne représentent pas simultanément -O- ou -S-, et
Y et Y¹ représente CI, F, CN, OCF₃ ou CF₃,
A³ et A⁴ représentent, chacun indépendamment de l'autre, de manière identique ou différente :
b) 1,4-phénylène, où un ou deux groupes CH peuvent être remplacés par N et où, en addition, un ou plusieurs atomes de H peuvent être remplacés par Br, CI, F, CN, méthyle, méthoxy ou un groupe méthyle ou méthoxy mono- ou polyfluoré,
ou
c) un radical pris parmi le groupe 1,4-bicyclo[2.2.2]octylène, pipéridine-1,4-diyle, cyclobutane-1,3-diyle, spiro-[3.3]heptane-2,6-diyle, 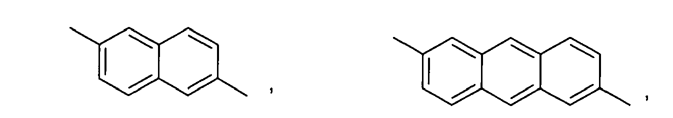 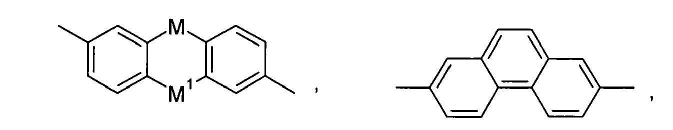 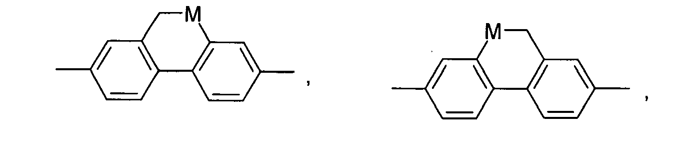 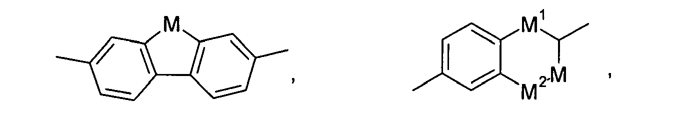 ou un ou plusieurs atomes a-nyarogene peuvent être substitués par F, CN, SCN, SF₅, CH₂F, CHF₂, CF₃, OCH₂F, OCHF₂ ou OCF₃,
une ou plusieurs liaisons doubles peuvent être remplacés par des liaisons simples, M, M¹ ou M² représente -O-, -S-, -CH₂-, -CHY- ou -CYY¹- de telle sorte que des groupes adjacents ne représentent pas simultanément -O- ou -S-, et Y et Y¹ représentent CI, F, CN, OCF₃ ou CF₃,
V représente H ou F,
Z¹ Z² et Z³ représentent, chacun indépendamment des autres, de manière identique ou différente une liaison simple, -CH₂O-, -(CO)O-, -CF₂O-, -CH₂CH₂CF₂O-, -CF₂CF₂-, -CH₂CF₂-, -CH₂CH₂-, -(CH₂)₄-, -CH=CH-, -CH=CF-, -CF=CF- ou -C≡C-, où des ponts asymétriques peuvent être orientés sur les deux côtés, et
a représente 0, 1 ou 2,
b représente 1 ou 2, et
c représente 0, 1 ou 2,
oùa+b+c<_4.

2. Composés selon la revendication 1 de la formule IA 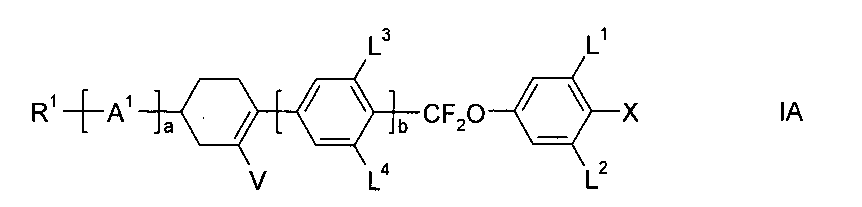 dans laquelle
R¹, A¹, a, b et V présentent les significations indiquées pour la formule I selon la revendication 1,
X représente F, OCF₃, CN, CF₃, SCN, SF₅, NCS, CI, OCHF₂, OCHFCF₃, OCF₂CHFCF₃,
V représente H ou F, et
L¹, L², L³ et L⁴ représentent, chacun indépendamment des autres, H ou F.

3. Composés selon la revendication 1 ou 2, **caractérisés en ce que**
R¹ représente alkyle, alcoxy, alkényle ou alkényloxy comportant jusqu'à 8 atomes de carbone.

4. Composés selon une ou plusieurs des revendications 1 à 3, **caractérisés en ce que** L¹ représente fluor et L² représente fluor ou hydrogène.

5. Composés selon une ou plusieurs des revendications 1 à 4 des formules I1 à I5, 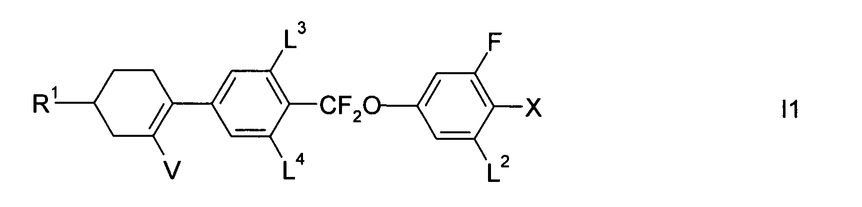 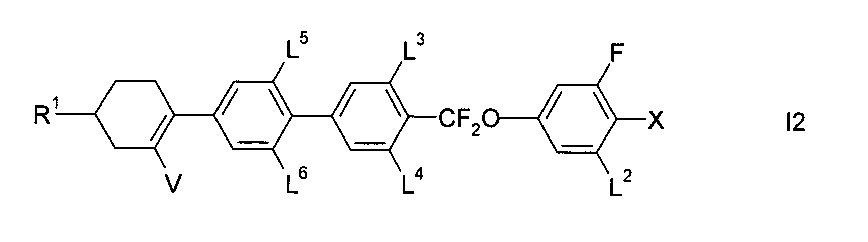 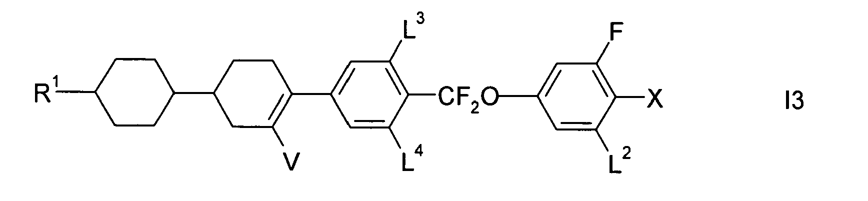 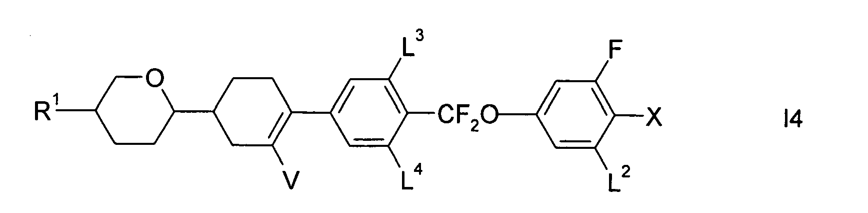 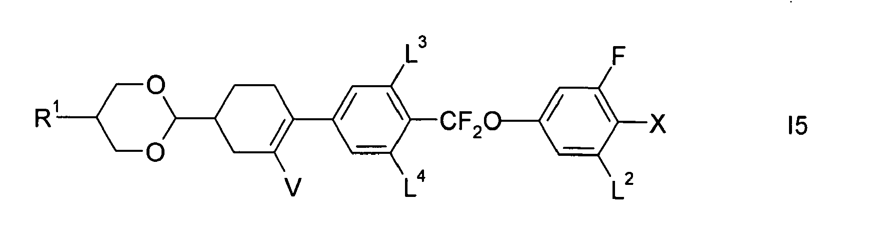 dans lesquelles R¹ et V présentent les significations indiquées selon la revendication 1, et
X représente F, OCF₃, CN, CF₃, SCN, SF₅, NCS, CI, OCHF₂, OCHFCF₃, OCF₂CHFCF₃, et
L², L³, L⁴, L⁵ et L⁶ représentent H ou F.

6. Composés selon une ou plusieurs des revendications 1 à 5, **caractérisés en ce que** L¹ et L² représentent fluor.

7. Composés selon une ou plusieurs des revendications 1 à 6, **caractérisés en ce que** V représente hydrogène.

8. Composés selon une ou plusieurs des revendications 1 à 6, **caractérisés en ce que** V représente fluor.

9. Procédé pour la préparation de composés de la formule I selon une ou plusieurs des revendications 1 à 7 dans lequel V représente hydrogène, comprenant une étape de procédé, **caractérisé en ce qu'**un cyclohexane cétone de la formule 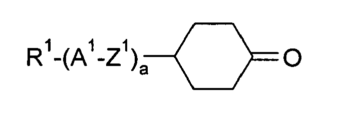 dans laquelle R¹, A¹, Z¹ et a sont comme défini dans la revendication 1,
est amené à réagir au niveau du groupe carbonyle avec un composé d'organomagnésium de la formule
MgHal-(Z²-A²)_{b}-CF₂O-(A³-Z_{c}-A⁴-R²
dans laquelle Z², Z³, A², A³, A⁴, a, c et R² sont comme défini dans la revendication 1, et
Hal représente Cl ou Br.

10. Procédé pour la préparation de composés de la formule 1 selon une ou plusieurs des revendications 1 à 8 dans lequel V représente fluor ou hydrogène, comprenant une étape de procédé, **caractérisé en ce qu'**un cyclohexène de la formule 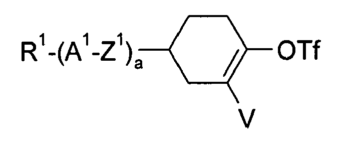 dans laquelle R¹, A¹, Z¹ et a sont comme défini dans la revendication 1,
est amené à réagir avec un acide boronique ou un ester d'acide boronique à chaîne ouverte ou cyclique des formules (OH)₂B-(Z²-A²)_{b}-CF₂O-(A³-Z³)_{c}-A⁴-R² 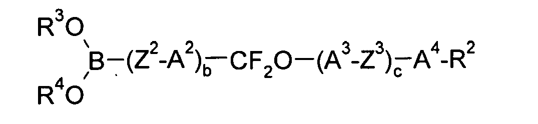 dans lesquelles Z², Z³, A², A³, A⁴, a, b et R² sont comme défini selon la revendication 1,
et
R³, R⁴ représentent alkyle comportant 1-12 atomes de C ou R³+R⁴ représentent ensemble également un alkylène, en particulier des formules
- CH₂-(CH₂)ₚ-CH₂- et -C(CH₃)₂C(CH₃)₂-,
ou 1,2-phénylène,
où R¹, R² et R¹+R² peuvent également être substitués et où p est 0 ou 1,
en présence d'un catalyseur à métal de transition.

11. Utilisation d'un ou de plusieurs composés de la formule 1 selon une ou plusieurs des revendications 1 à 8 en tant que composants dans un milieu cristallin liquide.

12. Milieu cristallin liquide comprenant au moins deux composés mésogènes, **caractérisé en ce qu'**il comprend au moins un composé de la formule I selon une ou plusieurs des revendications 1 à 8.

13. Utilisation du milieu cristallin liquide selon la revendication 12 à des fins électro-optiques.

14. Affichage à cristaux liquides électro-optique contenant un milieu cristallin liquide selon la revendication 12.
